(19) Europäisches Patentamt
European Patent Office
Office européen des brevets

(11) Veröffentlichungsnummer: **0 116 974**
**B1**

(12) # EUROPÄISCHE PATENTSCHRIFT

(45) Veröffentlichungstag der Patentschrift: **29.10.86**

(21) Anmeldenummer: **84101721.3**

(22) Anmeldetag: **20.02.84**

(51) Int. Cl.⁴: **C 07 J 41/00**, C 07 J 1/00, C 07 J 21/00, C 07 J 33/00, C 07 J 43/00, C 07 J 63/00, C 07 J 71/00, A 61 K 31/565, A 61 K 31/58

(54) **11-Beta-Aryl-Estradiene, Verfahren zu deren Herstellung und diese enthaltende pharmazeutische Präparate.**

(30) Priorität: **18.02.83 DE 3306121**
**18.02.83 DE 3306124**

(43) Veröffentlichungstag der Anmeldung: **29.08.84 Patentblatt 84/35**

(45) Bekanntmachung des Hinweises auf die Patenterteilung: **29.10.86 Patentblatt 86/44**

(84) Benannte Vertragsstaaten: **AT BE CH DE FR GB IT LI LU NL SE**

(56) Entgegenhaltungen:
**EP - A - 0 057 115**
**EP - A - 0 097 572**
**WO - A - 83/03099**
**DE - A - 2 748 250**
**FR - A - 2 244 497**
**FR - A - 2 342 990**
**FR - A - 2 377 418**
**GB - A - 2 017 708**
**GB - A - 2 073 201**
**US - A - 2 900 383**

**STEROIDS, Band 37, Nr. 4, April 1981, Sieten 361-382, San Francisco, USA A. BELANGER et al.: "Regio and stereospecific synthesis of 11 alpha-substituted 19-norsteroids"**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Neef, Günter, Dr., Darmstädter Strasse 9, D-1000 Berlin 15 (DE)**
Erfinder: **Sauer, Gerhard, Dr., Königsbacher Zeile 47 A, D-1000 Berlin 28 (DE)**
Erfinder: **Wiechert, Rudolf, Prof. Dr., Petzower Strasse 8 A, D-1000 Berlin 37 (DE)**
Erfinder: **Hofmeister, Helmut, Dr., Weislingenstrasse 4, D-1000 Berlin 28 (DE)**
Erfinder: **Rohde, Ralph, Dr., Schwatlowstrasse 4, D-1000 Berlin 45 (DE)**
Erfinder: **Annen, Klaus, Dr., Seegefelder Strasse 194, D-1000 Berlin 20 (DE)**
Erfinder: **Laurent, Henry, Dr., Glambecker Weg 21, D-1000 Berlin 28 (DE)**
Erfinder: **Beier, Sybille, Dr., Uhlandstrasse 121, D-1000 Berlin 31 (DE)**
Erfinder: **Losert, Wolfgang, Prof. Dr., Landshuter Strasse 22, D-1000 Berlin 30 (DE)**
Erfinder: **Elger, Walter, Dr., Schorlemer Allee 12 B, D-1000 Berlin 33 (DE)**
Erfinder: **Henderson, David Dr., Jahnstrasse 19, D-1000 Berlin 28 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 116 974 B1

ACTORUM AG

## Beschreibung

Die Erfindung betrifft neue 11β-Aryl-Estradiene, Verfahren zu deren Herstellung und diese Verbindung enthaltende pharmazeutische Präparate.

Die neuen 11β-Aryl-Estradiene werden durch die Formel I gekennzeichnet.

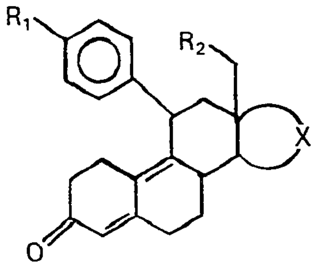

(I),

worin

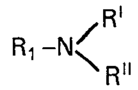 mit $R^I$ und $R^{II}$ in der Bedeutung von jeweils Alkyl mit 1 bis 4 Kohlenstoffatomen oder $R^I$ und $R^{II}$ unter Einschluss von N in der Bedeutung eines 5- oder 6gliedrigen heterocyclischen Ringes, wobei im Ring ausser N noch ein weiteres Heteroatom N, O oder S enthalten sein kann und die entsprechenden N-Oxide,

$-SR^{III}$ mit $R^{III}$ in der Bedeutung von Methyl, Ethyl oder Phenyl und

$-OR^{IV}$ mit $R^{IV}$ in der Bedeutung von Methyl, Ethyl, Propyl, Methoxymethyl, Allyl oder β-Dimethylaminoethyl,

$R_2$ ein Wasserstoffatom, eine Methyl- oder Ethylgruppe und

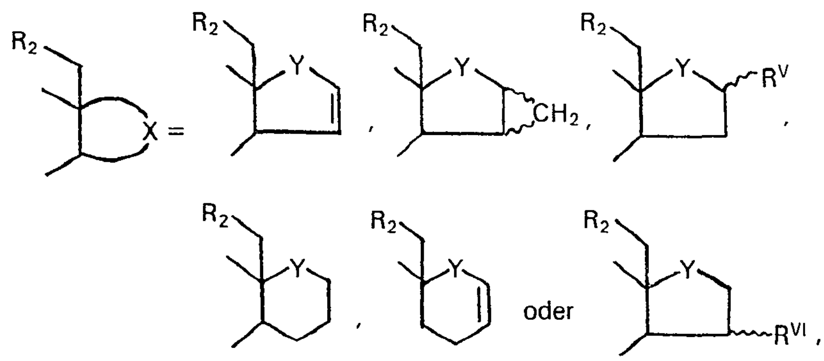

wobei

die Wellenlinien ∼∼ andeuten, dass der Substituent in α- oder β-Stellung und

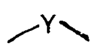 für 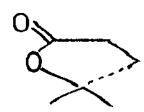, 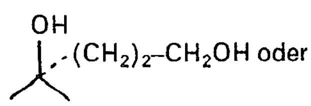 oder

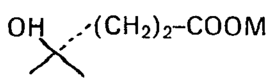 mit M = K, Na oder Li oder

für 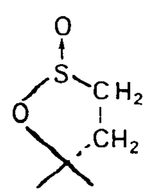, 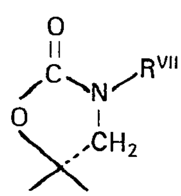 oder

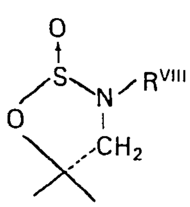

steht, und

$R^V$ und $R^{VI}$ und $R^{VII}$ und $R^{VIII}$ jeweils Alkyl mit 1 bis 4 Kohlenstoffatomen bedeuten.

11β-Aryl-Steroide sind bereits bekannt. So werden in EP-A-0 057 115, Steroids 37, 361 (1981), WO-A-8 303 099 und EP-A-0 097 572 derartige Verbindungen mit antiglucocorticoider und antigestagener sowie in FR-A-2 377 418 solche mit antiglucocorticoider und progestagener Wirkung beschrieben.

Aus GB-A-2 073 201, GB-A-2 017 708, FR-A-2 342 990, FR-A-2 244 497, USA-A-2 900 383 und DE-A-2 748 250 sind Steroide mit antimineralcorticoider Wirkung bekannt. Sie weisen jedoch eine andere Struktur als die erfindungsgemässen Verbindungen auf; so fehlt allen in den genannten Patentschriften beschriebenen Verbindungen der 11β-Aryl-Substituent sowie (mit Ausnahme der Verbindungen der letztgenannten Referenz) zusätzlich die 9, 10-Doppelbindung.

Es wurde nun gefunden, dass die neuen Verbindungen der allgemeinen Formel I überraschenderweise antigestagene und antimineralcorticoide Wirkungen besitzen, ohne dass nennenswerte antiglucocorticoide Wirkungen auftreten. Das gleichzeitige Auftreten von antigestagener und antimineralcorticoider Wirkung bei einer Substanz ist bisher noch nicht beschrieben worden. Überraschenderweise treten die beiden Effekte bei den neuen Verbindungen auch bei oraler Applikation in einer gegenüber den Verbindungen des Standes der Technik vergleichbaren Wirkungsstärke auf, wobei bei einigen erfindungsgemässen Verbindungen die antigestagene und bei anderen die antimineralcorticoide Wirkung überwiegt. Die neuen Verbindungen der allgemeinen Formel I sind damit prinzipiell sowohl zur Fertilitätskontrolle als auch zur Behandlung von Krankheitszuständen geeignet, an denen ein Hyperaldosteronismus beteiligt ist.

Die antimineralcorticoide Wirkung wurde im Antialdosterontest bestimmt. In diesem Test wurden adrenalektomierte, mit Fluocortolon und Fluocortoloncapronat substituierte, nüchterne Wistar-Ratten mit 1,0–2,0–4,0 mg Prüfsubstanz pro Tier behandelt. Die Prüfsubstanz wurde als Kristallsuspension in NaCl/Myrj 53 oral appliziert. Eine Stunde nach der Applikation erhielten die Tiere eine intravenöse Dauerinfusion von physiologischer Kochsalzlösung mit einem Zusatz von 0,14 µg Aldosteron pro Tier pro Stunde. Von der dritten bis zur zehnten Stunde wurden stündlich Na- und K-Salzausscheidung gemessen, und es wurden der Na/K-Quotient sowie der Quotient log Na (100)/K ermittelt.

Als Prüfsubstanzen dienten das erfindungsgemässe 11β-(4-Dimethylaminophenyl)-17β-hydroxy-17α-(3-hydroxypropyl)-4,9(10)-estradien-3-on (A) und das bekannte Spironolakton (B).

In der folgenden Tabelle 1 werden die Na/K- und log Na (100)/K-Quotienten sowie die relativen Antialdosteronwirkungen (RW) von A bezogen auf B = 1 zusammengestellt.

Tabelle 1

Antialdosterontest

| Std. | Na/K A (mg) 1.0 | A 2.0 | A 4.0 | B (mg) 1.0 | B 2.0 | B 4.0 | RW | log Na (100)/K A (mg) 1.0 | A 2.0 | A 4.0 | B (mg) 1.0 | B 2.0 | B 4.0 | RW |
|---|---|---|---|---|---|---|---|---|---|---|---|---|---|---|
| 3. | 2.33 | 5.66 | 6.54 | 3.30 | 5.39 | 6.23 | 0.95 | 2.30 | 2.70 | 2.71 | 2.49 | 2.70 | 2.71 | 0.76 |
| 4. | 0.91 | 2.86 | 4.19 | 1.73 | 2.19 | 4.53 | 0.91 | 1.90 | 2.42 | 2.53 | 2.04 | 2.26 | 2.49 | 1.04 |
| 5. | 0.82 | 2.11 | 3.21 | 0.99 | 1.69 | 3.37 | 1.02 | 1.82 | 2.28 | 2.37 | 1.83 | 2.13 | 2.34 | 1.17 |
| 6. | 0.76 | 1.79 | 3.14 | 0.94 | 1.65 | 3.31 | 0.96 | 1.73 | 2.18 | 2.37 | 1.75 | 2.08 | 2.31 | 1.12 |
| 7. | 0.89 | 1.55 | 3.31 | 0.84 | 1.57 | 3.64 | 0.95 | 1.89 | 2.16 | 2.39 | 1.76 | 2.06 | 2.37 | 1.22 |
| 8. | 0.84 | 1.02 | 3.50 | 0.51 | 1.11 | 3.37 | 1.07 | 1.77 | 1.94 | 2.37 | 1.50 | 1.89 | 2.34 | 1.27 |
| 9. | 0.82 | 0.96 | 3.87 | 0.65 | 0.97 | 3.33 | 1.07 | 1.81 | 1.93 | 2.37 | 1.57 | 1.80 | 2.33 | 1.29 |
| 10. | 0.96 | 1.09 | 2.84 | 0.40 | 1.08 | 2.63 | 1.16 | 1.89 | 1.96 | 2.30 | 1.45 | 1.85 | 2.21 | 1.60 |

Die Tabelle zeigt, dass die erfindungsgemässe Verbindung A etwa gleiche Wirksamkeit besitzt wie Spironolakton.

Zur Kennzeichnung der antigestagenen Wirkung wurde die abortive Wirkung bestimmt.

Die Versuche wurden an weiblichen Ratten im Gewicht von ca. 200 g durchgeführt. Nach erfolgter Anpaarung wurde der Schwangerschaftsbeginn durch Nachweis von Spermien in Vaginalabstrichen gesichert. Der Tag des Spermiennachweises gilt als Tag 1 der Gravidität (= d1 p.c.).

Die Behandlung der Tiere mit der jeweils zu testenden Substanz bzw. dem Lösungsmittel erfolgte nach der Nidation der Blastocysten von d5 p.c. bis d7 p.c. An d9 p.c. wurden die Tiere getötet und die Uteri auf Implantate und Resorptionsstellen hin untersucht. Von allen Uteri wurden Fotos angefertigt. Das Fehlen von Implantaten wurde als Abort gewertet.

Die Testsubstanzen wurden in einem Benzylbenzoat-Rizinusöl-Gemisch (Verhältnis 1+9) gelöst. Das Vehikelvolumen pro Einzeldosis betrug 0.2 ml. Die Behandlung erfolgte subcutan.

Testsubstanzen waren die erfindungsgemässe Verbindung A und das in der europäischen Patentanmeldung 82 400 025.1 beschriebene 11β-(4-Dimethylaminophenyl)- 17β- hydroxy- 17α-(propin -1 - yl)-4,9(10)-estradien -3- on (C).

Tabelle 2

Abortivtest bei der graviden Ratte

| Substanz | Dosis mg/Tier/Tag s.c. | n Abort-positiv / n Gesamt |
|---|---|---|
| A | 10,0 | 4/4 |
| | 3,0 | 4/4 |
| | 1,0 | 0/4 |
| C | 3,0 | 4/4 |
| | 1,0 | 2/4 |
| | 0,3 | 0/4 |

Aus Tabelle 2 ist zu entnehmen, dass sowohl die erfindungsgemässe Verbindung A als auch die bekannte Verbindung C bei einer Dosis von 3,0 mg abortiv voll wirksam ist.

Die erfindungsgemässen Verbindungen besitzen im Gegensatz zur bekannten Verbindung C keine nennenswerte antiglucocorticoide, sondern antimineralcorticoide Nebenwirkung. Bei der Anwendung der erfindungsgemässen Verbindungen als Antigestagene zur Aufhebung von Schwangerschaften bzw. zur frühen Auslösung einer Menstruation ist eine antimineralcorticoide Wirkung nicht schädlich und in Fällen des Hyperaldosteronismus sogar erwünscht.

Die Behandlung von bestimmten Formen des Aldosteronismus, der Hypertonie, von Ödemen und anderer durch Aldosteron bedingter Störungen kann auch das Hauptanwendungsgebiet der Verbindungen der allgemeinen Formel I sein.

Die Erfindung betrifft auch pharmazeutische Präparate, die Verbindungen der allgemeinen Formel I enthalten.

Die pharmakologisch wirksamen erfindungsgemässen Verbindungen der allgemeinen Formel I können nach an sich bekannten Methoden der Galenik zu pharmazeutischen Präparaten für die orale oder parenterale Applikation verarbeitet werden.

Die Dosierung der erfindungsgemässen Verbindungen liegt beim Menschen bei 10 bis 1000 mg pro Tag.

In den Verbindungen der allgemeinen Formel I stellt der Arylrest in 11β-Stellung des Steroidgerüstes einen durch $R_1$ in para-Stellung substituierten Phenylrest dar. Wenn $R_1$ für $N\langle{}^{R^I}_{R^{II}}$ steht, bedeuten $R^I$ und $R^{II}$ Alkylgruppen mit 1 bis 4 Kohlenstoffatomen, wobei die Methyl- und die Ethylgruppe bevorzugt sind. Die Gruppe $N\langle{}^{R^I}_{R^{II}}$ steht auch fur einen heterocyclischen Fünf- oder Sechsring, der ausser N- und C-Atome auch noch zusätzlich ein O- oder S-Atom enthalten kann; beispielsweise genannt seien der Pyrrolidino-, Piperidino-, Piperazino-, Morpholino-, Oxa- und Thiazolidino- sowie Thiadiazolidinoring. Unter $N\langle{}^{R^I}_{R^{II}}$ sollen auch die entsprechenden N-Oxide verstanden werden, wie zum Beispiel Dimethylamino-N-Oxid, Pyrrolidino-, Piperidino-, Piperazino- usw. N-Oxid.

Der in Formel I durch $R^V$, $R^{VI}$, $R^{VII}$ und $R^{VIII}$ dargestellte Rest ist ein Alkylrest mit 1 bis 4 Kohlenstoffatomen, vorzugsweise der Methyl- oder Ethylrest.

Die neuen Verbindungen der allgemeinen Formel I werden erfindungsgemäss nach dem Verfahren gemäss Anspruch 20 hergestellt.

Ausgehend von den 17α-Oxypropyl-17β-hydroxy-Verbindungen der allgemeinen Formel II wird zur Wasserabspaltung unter Ausbildung der 4(5)-Doppelbindung und zur gleichzeitigen Ketalspaltung und Entfernung gegebenenfalls vorhandener weiterer mit Säure abspaltbarer Schutzgruppen mit Säure oder einem sauren Ionenaustauscher behandelt.

Die saure Behandlung erfolgt in an sich bekannter Weise, indem man die Verbindung der Formel II, die eine 3-Ketalgruppe und eine 5α-Hydroxygruppe und eine gegebenenfalls O-geschützte 17α-(3-hydroxypropyl)-Gruppe enthält, in einem mit Wasser mischbaren Lösungsmittel, wie wässrigem Methanol, Ethanol oder Aceton, löst und auf die Lösung katalytische Mengen Mineral- oder Sulfonsäure, wie Salzsäure, Schwefelsäure, Phosphorsäure, Perchlorsäure oder p-Toluolsulfonsäure oder eine organische Säure, wie Essigsäure, so lange einwirken lässt, bis Wasser abgespalten ist und Schutzgruppen entfernt sind. Die Umsetzung, die bei Temperaturen von 0 bis 100 °C abläuft, kann auch mit einem sauren Ionenaustauscher vorgenommen werden. Der Verlauf der Umsetzung kann mit analytischen Methoden, beispielsweise durch Dünnschichtchromatographie entnommener Proben, verfolgt werden.

Werden Endprodukte der Formel I gewünscht, in denen Y für 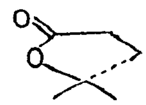 steht, so werden die 17α-(3-Hydroxypropyl)-Verbindungen (Y = 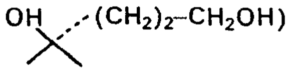) in an sich bekannter Weise oxydiert. Die Bedingungen bei der Oxydation sind abhängig von der Natur des Substituenten $R_1$ in Formel I. Der Fachmann wird von Fall zu Fall entscheiden, welche der bekannten Methoden die geeignete ist und welche nicht in Frage kommt. Ist $R_1$ zum Beispiel ein Dialkylamino- oder Alkylthiorest, so sind Chromsäurereagenzien zur Oxidation ungeeignet, da diese primär an der Dialkylamino- oder Alkylthiogruppe angreifen. In diesen Fällen müssen Oxidationsmittel, wie Silbercarbonat/Celite (Fetizon-Reagenz; M. Fetizon und M. Golfier, Compt. rend. 267 (1968) 900) oder Platin/Sauerstoff (H. Muxfeldt et al., Angewandte Chemie, Int. Ed. 1 (1962) 157) Verwendung finden. Ist $R_1$ dagegen eine Alkoxygruppe, so können auch Oxydationsmittel, wie Jones' Reagenz, Chromsäure-Pyridin, Pyridiniumdichromat oder Pyridiniumchlorochromat verwendet werden.

Werden Endprodukte der Formel I gewünscht, in denen Y für

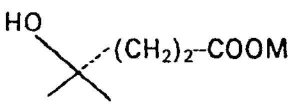 mit M = Alkalimetall

steht, so werden die 17-Spirolaktone (Y =

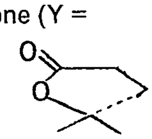 )

ebenfalls in bekannter Weise umgesetzt. Hierzu wird das Lakton mit verdünnter Alkalilauge behandelt, wobei sich das Alkalisalz der 17α-Propionsäure bildet. Alkalisalze sind Kalium-, Natrium- oder Lithiumsalze, wobei das Kaliumsalz bevorzugt ist.

Ausgehend von den Oxiranen der allgemeinen Formel II, die aus den entsprechenden 17-Ketonen mit Trimethylsulfoniumjodid hergestellt werden, wird zunächst der 17-Spiro-Heterocyclus nach b) und c) bzw. nach a) eine Vorstufe des Heterocyclus [21-(1,1-Dimethyl-sulfinyl)] gebildet.

Nach a) erfolgt die Umsetzung der Verbindung der allgemeinen Formel II mit tert. Butylmethylsulfoxid und Alkyllithium in Tetrahydrofuran. Als Alkyllithium sind zum Beispiel Methyl- und n-Butyllithium geeignet.

Nach b) wird die Verbindung der allgemeinen Formel II zunächst mit Alkylamin, insbesondere Methyl- oder Ethylamin, in Gegenwart von Säure, wie p-Toluolsulfonsäure, auf 80 bis 150 °C erhitzt. Die so erhaltene 17α-Alkylaminomethyl-Verbindung wird anschliessend mit Kohlensäuredialkylester, insbesondere -dimethyl- oder diethylester, in Gegenwart einer Base, wie Kalium-tert.-butylat oder Alkali-propionat oder -phenylat, unter Rückfluss erhitzt. Nach dieser Methode wird das [17(β-1')-Spiro-5']-oxazolidin -2- on unter gleichzeitiger Wasserabspaltung und Ausbildung der 4(5)-Doppelbindung erhalten.

Nach einer anderen Methode wird die Verbindung der allgemeinen Formel II mit N-Alkylurethan und Kalium-tert.-butylat in Hexamethylphosphorsäuretriamid zum [17(β-1')-Spiro-5']-oxazolidin -2- on umgesetzt. Die Umsetzung erfolgt bei Temperaturen von etwa 80 bis 150 °C. Als N-Alkylurethan sind N-Methyl- und N-Ethylurethan besonders geeignet.

Nach c) wird die Verbindung der allgemeinen Formel II zunächst mit Alkylamin, insbesondere Methyl- oder Ethylamin, in Gegenwart von Säure, wie p-Toluolsulfonsäure, auf 80 bis 150 °C erhitzt. Die so erhaltene 17α-Alkylaminomethyl-Verbindung wird anschliessend bei 0 bis 20 °C mit Thionylchlorid in Gegenwart von Triethylamin behandelt und liefert [17(β-1')-Spiro-5'] [1,2,3]-oxathiazolidin -2- oxid.

Zur anschliessenden Wasserabspaltung aus der 5α-Hydroxyverbindung unter Ausbildung der 4(5)-Doppelbindung und gleichzeitiger Ketalspaltung lässt man nach a), b) und c) eine wässrige Säure, beispielsweise wässrige Essigsäure, bei Temperaturen von 0 bis 100 °C einwirken.

Der Ringschluss zum [17(β-1')-Spiro-5'] [1,2]-Oxathiolan -2- Oxid erfolgt nach a) durch Umsetzung der 21-(1,1-Dimethylsulfinyl)-Verbindung

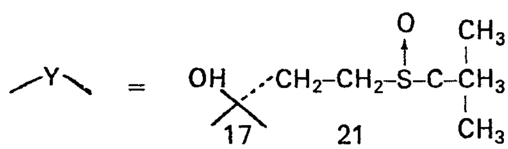

mit N-Chlor- oder N-Bromsuccinimid in Gegenwart von Wasser.

Die zur Durchführung des erfindungsgemässen Verfahrens benötigten Ausgangsverbindungen der allgemeinen Formel II mit

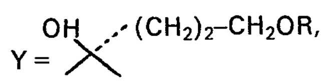

wobei R ein Wasserstoffatom oder eine im sauren Milieu leicht abspaltbare Gruppe darstellt, können nach bekannten Verfahren hergestellt werden. Als leicht abspaltbare Gruppen kommen Alkoxyalkylgruppen, wie Methoxymethyl, Ethoxy-

methyl, oder ringgeschlossene Ether, wie Tetrahydropyran, infrage.

Die Herstellung der Ausgangsverbindungen der allgemeinen Formel II soll anhand des folgenden Reaktionsschemas erläutert werden:

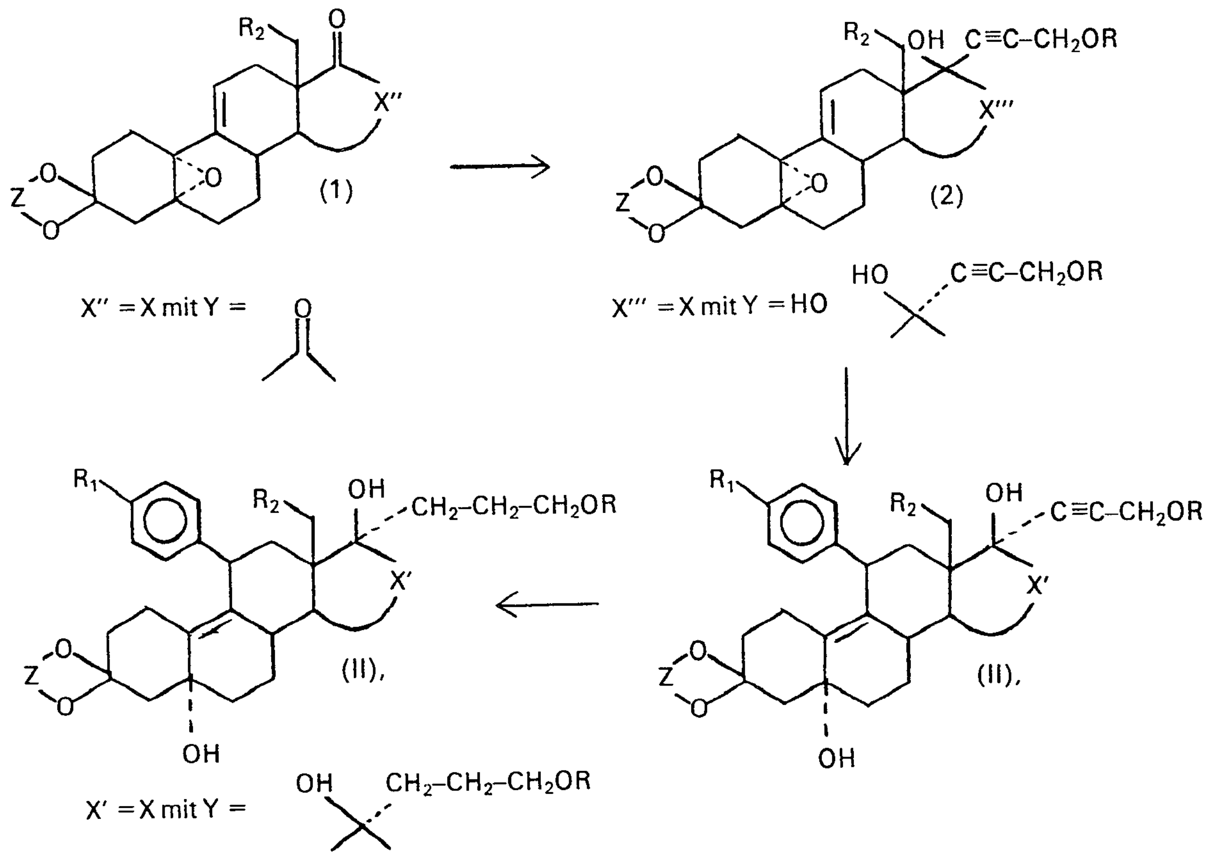


Oxirane (1) gemäss europäischer Patentanmeldung Nr. 82 400 025.1 (Publikations-Nr. 0 057 115) werden mit metallierten Derivaten des Propargylalkohols, zum Beispiel mit 1-Lithium-3-tetrahydropyran-2'-yloxypropin-1, zu den 17α-[3-oxygenierten -1- propinyl]-17β-hydroxy-Verbindungen (2) umgesetzt. Die Einführung des 11β-Arylrestes zu (3) erfolgt entweder durch Cu(I)-katalysierte Grignard-Reaktion mit den entsprechenden Arylmagnesiumhalogeniden (Tetrahedron Letters 1979, 2051) oder durch Umsetzung mit gemischten Organocupraten des Typs $R_2Cu(CN)Li_2$ (J. Am. Chem. Soc. 103 (1981) 7672). Die Hydrierung von (3) zu den Ausgangsverbindungen (II) muss unter Bedingungen durchgeführt werden, die ausschliesslich den Angriff an der C-C-Dreifachbindung gewährleisten, ohne die tetrasubstituierte 9(10)-Doppelbindung abzusättigen. Das gelingt zum Beispiel, wenn bei Raumtemperatur und Normaldruck in Lösungsmittel, Methanol, Ethanol, Propanol, Tetrahydrofuran (THF) oder Essigester, unter Zusatz von Edelmetall-Katalysatoren, wie Platin oder Palladium, hydriert wird.

Im folgenden wird die Herstellung einiger Ausgangsverbindungen der allgemeinen Formel II näher erläutert:

1 a) Zu einer Lösung von 35,7 g 3-Tetrahydropyran-2'-yloxy -1- propin in 760 ml absolutem Tetrahydrofuran tropft man unter Eiswasserkühlung 208 ml einer 15%igen Lösung von n-Butyllithium in Hexan. Nach Zugabe rührt man weitere 15 Minuten bei +5 bis +10 °C und gibt anschliessend tropfenweise eine Lösung von 23,7 g 3,3-(2,2-Dimethyl-propan-1,3-dioxy)-5α, 10α-oxido-9(11)-estren -17- on in 470 ml absolutem THF hinzu. Man rührt danach 20 Minuten bei 25 °C, giesst daraufhin die Reaktionslösung in ca. 5 l Eiswasser und extrahiert mit Essigester. Der Essigesterextrakt wird über Natriumsulfat/Aktivkohle getrocknet und im Vakuum eingeengt. Nach Filtration über Aluminiumoxid mit Hexan/Essigester als Elutionsmitteln erhält man 29,3 g 3,3-(2,2-Dimethyl-propan-1,3-dioxy)-5α, 10α-oxido-17α-[3-(tetrahydropyran 2-yloxy) -1-propinyl]-9(11)-estren-17β-ol als farbloses Öl.

b) Eine Suspension von 5,28 g Magnesium (Späne) in 275 ml absoluten THF wird sukzessive mit 0,05 ml Methyljodid und einer Lösung von 50,27 g 4-Bromdimethylanilin in 245 ml absolutem THF versetzt. Man rührt bis zur vollständigen Auflösung des Magnesiums in einer Argon-Atmosphäre, wobei die Innentemperatur 50 °C nicht übersteigen soll. Anschliessend kühlt man auf +5 °C, versetzt die Grignard-Lösung mit 1,12 g CuCl und rührt 15 Minuten bei +5 – +10 °C. Im Anschluss daran wird eine Lösung von 29,3 g des unter a) erhaltenen Produkts in 275 ml absolutem

THF hinzugetropft und 5 Stunden bei Raumtemperatur nachgerührt. Dann wird die Reaktionslösung in ca. 4 l Eiswasser gegossen und mit Essigester extrahiert. Die Chromatographie des so erhaltenen Rohprodukts über Aluminiumoxid mit Hexan/Essigester ergibt 32,6 g 11β-(4-Dimethylaminophenyl) -3,3- (2,2- dimethylpropan-1,3-dioxy)-17α-[3-(tetrahydropyran -2- yloxy)-1- propinyl]-9(10)-estren-5α, 17β-diol als gelbliches Öl.

c) Eine Lösung von 2,0 g des unter b) erhaltenen Produkts in 20 ml Ethanol wird nach Zusatz von 120 mg Palladiumkohle (10%) bei Raumtemperatur und Normaldruck hydriert. Nach 2 Stunden und einer $H_2$-Aufnahme von 141 ml filtriert man vom Katalysator ab und engt das Filtrat ein. Das so gewonnene Rohprodukt wird ohne weitere Reinigung in Beispiel 1 eingesetzt.

2 a) Gemäss 1 a) setzt man 22,6 g 3,3-(2,2-Dimethylpropan-1,3-dioxy)-18-methyl-5α, 10α-oxido-9(11)-estren -17- on (Schmelzpunkt 156–158 °C) mit 1-Lithio -3- (tetrahydropyran-2- yloxy)-propin-1 um und erhält 25,4 g 3,3-(2,2-Dimethylpropan-1,3-dioxy) -18- methyl-5α, 10α-oxido-17α-[3-(tetrahydropyran -2- yloxy) -1- propinyl]-9(11)-estren-17β-ol als farbloses Öl.

b) Zu einer Suspension von 4,3 g Magnesium (Späne) in 40 ml absolutem THF tropft man bei 25 °C zunächst 0,1 ml Methyljodid, dann eine Lösung von 40 g p-Dimethylaminoethoxy-phenylbromid (hergestellt nach D. Lednicer et al., J. Med. Chem. 8, 52 (1965) ) in 200 ml absolutem THF. Man rührt bis zur vollständigen Auflösung des Magnesiums bei einer Badtemperatur von maximal 70 °C. Nach dem Abkühlen auf 0 °C gibt man 820 mg CuCl hinzu und rührt 20 Minuten bei 0 °C. Sodann wird eine Lösung von 15,9 g des unter a) erhaltenen Produkts in 120 ml absolutem THF hinzugetropft. Man rührt 16 Stunden bei 25 °C, giesst in Eiswasser und extrahiert mit Essigester. Die Chromatographie über Aluminiumoxid (neutral, III) mit Hexan/Essigester ergibt 17,1 g gelbliches Öl.

c) Die Hydrierung von 3,4 g des unter b) erhaltenen Produkts nach 1 c) ergibt 3,4 g 11β-[4-(2-Dimethylaminoethoxy)-phenyl]-3,3-(2,2-dimethyl-propan-1,3-dioxy) -18- methyl- 17α-[3-(tetrahydropyran -2- yloxy)-propyl]-9(10)-estren-5α, 17β-diol.

3 a) Aus 2,4 g Magnesium in 120 ml absolutem THF und 11,6 ml 4-Bromanisol wird wie unter 1 b) beschrieben ein Grignard-Reagenz hergestellt und mit 260 mg CuCl versetzt. Eine Lösung von 6,4 g des unter 1 a) hergestellten Addukts in 80 ml absolutem THF wird bei 0 °C hinzugetropft. Die Reaktionslösung wird 4 Stunden bei 25 °C gerührt und wie unter 1 b) aufgearbeitet. Man erhält 7,15 g öliges Produkt.

b) 7,15 g des unter a) erhaltenen Produkts werden wie unter 1 c) hydriert. Man erhält 7,05 g Rohprodukt, das ohne weitere Reinigung in Beispiel 3 weiter verwendet wird.

4 a) Aus 6,7 g Propargyl-tetrahydropyranyläther in 100 ml THF und 40 ml n-Butyllithium (15% in Hexan) wird unter den Bedingungen von 1 a) die lithiumorganische Verbindung hergestellt und mit 4,63 g 3,3-(2,2-Dimethyl-propan-1,3-dioxy)-16β-methyl-5α, 10α-oxido-9(11)-estren-17- on umgesetzt. Nach Chromatographie erhält man 4,22 g 3,3-(2,2-Dimethyl-propan-1,3-dioxy)-16β-methyl-5α, 10α-oxido-17α-[3-(tetrahydropyran -2- yloxy)-prop -1- inyl]-9(11)-estren-17β-ol als kristallines Isomerengemisch vom Schmelzpunkt 156–166 °C.

b) Aus 1,23 g Magnesiumspänen in 100 ml absolutem THF 11,48 g 4-Dimethylaminophenylbromid in 50 ml absolutem THF, 0,03 ml Methyljodid und 230 mg CuCl wird unter den Bedingungen von 1 b) ein Grignard-Reagenz hergestellt und mit 3,55 g 3,3-(2,2-Dimethyl-propan-1,3-dioxy)-16β-methyl-5α, 10α-oxido-17α-(3-tetrahydropyran -2- yloxy-prop -1- inyl)-9(11)-estren- 17β-ol umgesetzt. Man erhält nach Chromatographie 3,56 g 11β-(4-Dimethylaminophenyl)- 3,3- (2,2-dimethylpropan- 1,3-dioxy-16β-methyl- 17α- [3-(tetrahydropyran -2- yloxy)-prop- 1- inyl]-9(10)-estren-5α, 17β-diol als öliges Isomerengemisch.

c) Hydrierung von 3,56 g des Produkts der Grignard-Reaktion unter den Bedingungen von 1 c) ergibt 3,42 öliges 11β-(4-Dimethylaminophenyl)- 3,3-(2,2-dimethyl-propan-1,3-dioxy)-16β-methyl-17α-[3-(tetrahydropyran -2- yloxy)-propyl]-9(10)-estren-5α, 17β-diol.

5 a) Zu einer Suspension von 1,02 g Magnesiumspänen und 0,05 ml Methyljodid in 25 ml absolutem Tetrahydrofuran wird eine Lösung von 10,5 g 4-(2,5-Dimethylpyrrol -1- yl) brombenzol (hergestellt nach J.Chem. Soc. 195, 3155) in 40 ml absolutem Tetrahydrofuran in der Weise getropft, dass die Temperatur nach Anspringen der Reaktion 45 °C nicht überschreitet. Nach Auflösung des Magnesiums kühlt man auf 0 °C, gibt 210 mg CuCl hinzu, rührt 15 Minuten bei 0 °C und tropft schliesslich eine Lösung von 4,00 g 3,3-(2,2-Dimethylpropan- 1,3-dioxy)-5α, 10α-oxido-17α- [3-(tetrahydropyran -2- yloxy)prop- 1-inyl]-9(11)-estren-17β-ol in 50 ml absolutem Tetrahydrofuran zu. Anschliessend lässt man über Nacht bei Raumtemperatur rühren, giesst das Reaktionsgemisch auf Eiswasser und extrahiert mit Essigester. Durch Chromatographie an $Al_2O_3$ (neutral, III) mit Hexan/Essigester erhält man 4,42 g 3,3- (2,2-Dimethylpropan-1,3-dioxy)-11β-[4-(2,5-Dimethylpyrrol -1- yl)phenyl]-17α-[3-(tetrahydropyran -2- yloxy)-prop- 1- inyl]-9(10)-estren-5α, 17β-diol als blassgelben festen Schaum.

b) 4,15 g des unter a) erhaltenen Produkts werden in 50 ml Ethanol unter Zusatz von 400 mg Palladiumkohle (10%) unter Normalbedingungen hydriert. Nach Aufnahme von 290 ml $H_2$ wird vom Katalysator abfiltriert und eingeengt. Durch Chromatographie an $Al_2O_3$ (neutral, III) mit Hexan/Essigester erhält man 3,37 g 3,3-(2,2-Dimethylpropan- 1,3-dioxy)- 11β- [4- (2,5-dimethylpyrrol -1- yl) phenyl]-17α-[3-(tetrahydropyran -2- yloxy)propyl]-9(10)-estren-5α, 17β-diol als blassgelben festen Schaum.

6 a) 1,46 g Magnesiumspäne und 0,05 ml Methyliodid werden in 15 ml absolutem Tetrahydrofuran vorgelegt. Anschliessend tropft man eine Lösung von 14,5 g N-(4-Bromphenyl)piperidin (hergestellt nach J.Am.Chem.Soc. 75, 5280 (1953) ) in 100 ml absolutem Tetrahydrofuran in der Weise zu, dass die Temperatur nach Anspringen der Reaktion 45 °C nicht überschreitet. Nach Auflösung des Magnesiums kühlt man auf 0 °C, gibt 450 mg CuCl hinzu, rührt 15 Minuten bei 0 °C und tropft schliesslich eine Lösung von 6,0 g 3,3-(2,2-Dimethylpropan-1,3-dioxy)-5α, 10α-oxido-17α-[3-(tetrahydropyran -2- yloxy)prop-1- inyl]-9(11)-estren- 17β-ol in 50 ml absolutem Tetrahydrofuran zu. Anschliessend lässt man über Nacht bei Raumtemperatur rühren, giesst das Reaktionsgemisch auf Eiswasser und extrahiert mit Essigester. Durch Chromatographie an $Al_2O_3$ (neutral, III) mit Hexan/Essigester erhält man 7,0 g 3,3-(2,2-Dimethylpropan-1,3-dioxy)-11β-(4-piperidinophenyl)- 17α- [3-(tetrahydropyran- 2- yloxy)prop -1- inyl]-9(10)-estren-5α, 17β- diol als farblosen festen Schaum.

b) 7,0 g des unter a) erhaltenen Produkts werden in 250 ml Ethanol unter Zusatz von 680 mg Palladiumkohle (10%) unter Normalbedingungen hydriert. Nach Aufnahme von 470 ml $H_2$ wird vom Katalysator abfiltriert und eingeengt. Man erhält so 6,86 g 3,3-(2,2-Dimethylpropan-1,3-dioxy)- 11β- (4-piperidinophenyl)- 17α-[3- (tetrahydropyran -2- yloxy)propyl]-9(10)-estren-5α, 17β-diol als farbloses Öl, welches ohne weitere Aufreinigung in der folgenden Stufe eingesetzt wird (Beispiel 8).

7 a) 2,16 g Magnesiumspäne und 0,05 ml Methyljodid werden in 15 ml absolutem Tetrahydrofuran vorgelegt. Anschliessend tropft man eine Lösung von 13,5 g N-(4-Bromphenyl)pyrrolidin (hergestellt nach J.Am.Chem.Soc. 75, 5280 (1953) ) in 150 ml absolutem Tetrahydrofuran in der Weise zu, dass die Temperatur des Reaktionsgemisches nach Anspringen der Reaktion 45 °C nicht überschreitet. Nach Auflösung des Magnesiums kühlt man auf 0 °C, gibt 450 mg CuCl hinzu, rührt 15 Minuten bei 0 °C und tropft schliesslich eine Lösung von 6,0 g 3,3-(2,2-Dimethyl-propan-1,3-dioxy)-5α, 10α-oxido-17α-[3-(tetrahydropyran -2- yloxy)-prop -1- inyl]-9(11)-estren-17β-ol in 70 ml absolutem Tetrahydrofuran zu. Anschliessend lässt man über Nacht bei Raumtemperatur rühren, giesst das Reaktionsgemisch auf Eiswasser und extrahiert mit Essigester. Durch Chromatographie an $Al_2O_3$ (neutral, III) mit Hexan/Essigester erhält man so 7,0 g 3,3-(2,2-Dimethylpropan-1,3-dioxy)-11β-[4-(pyrrolidin -1- yl)phenyl]-17α- [3-(tetrahydropyran -2- yloxy)prop -1- inyl]-9(10)-estren-5α, 17β-diol als farblosen festen Schaum.

b) Eine Lösung von 7,0 g des unter a) erhaltenen Produkts in 250 ml Ethanol wird nach Zusatz von 670 mg Palladiumkohle (10%) bei Raumtemperatur und Normaldruck hydriert. Nach einer Aufnahme von 470 ml $H_2$ filtriert man vom Katalysator ab, engt das Filtrat ein und chromatographiert an $Al_2O_3$ (neutral, III) mit Hexan/Essigester. Man erhält so 6,3 g 3,3-(2,2-Dimethylpropan-1,3-dioxy)-11β-[4-(pyrrolidin -1- yl)phenyl]-17α-[3-(tetrahydropyran -2- yloxy)propyl]-9(10)-estren-5α, 17β-diol als farblosen Schaum.

Beispiel 1

3- [11β- (4-Dimethylaminophenyl)- 17β- hydroxy -3- oxo-4,9(10)-estradien-17α-yl]-propionsäure-lakton

1,96 g 11β-(4-Dimethylaminophenyl)-3,3-(2,2-dimethylpropan- 1,3-dioxy)-17α-[3-(tetrahydropyran -2- yloxy)-propyl]-9(10)-estren-5α, 17β-diol werden in 19 ml 70%iger wässriger Essigsäure aufgenommen und 3 Stunden bei 60 °C gerührt. Anschliessend giesst man in Eiswasser, stellt durch Zugabe von konzentrierter wässriger $NH_3$-Lösung einen pH-Wert von ca. 10 ein und extrahiert mit Methylenchlorid. Nach Filtration des Rohprodukts über Kieselgel (Hexan/Essigester) erhält man 1,15 g 11β-(4-Dimethylaminophenyl)- 17β-hydroxy- 17α- (3-hydroxypropyl)-4,9(10)-estradien -3- on als festen Schaum.

$^1H$–NMR ($CDCl_3$) : δ = 0,60 ppm (δ, 3H, H–18), 2,92 (δ, 6H, $N(CH_3)_2$; 3,71 (m, 2H, $CH_2OH$); 4,34 (m, 1H, H–11); 5,77 (m, 1H, H-4); 6,66 und 7,00 (AA'BB'-System, je 2H, arom. H). UV (MeOH): λ 260 nm (ε = 16 990); 304 (19 720).

Eine Lösung von 1,0 g obigen Produkts in 150 ml Toluol wird nach Zusatz von 7,45 g Silbercarbonat auf Celite (hergestellt nach M. Fetizon and M. Golfier, Compt. rend., 1968, 267, 900) 6 Stunden unter Rückfluss erhitzt. Nach dem Abkühlen filtriert man, wäscht den Filterrückstand gründlich mit Methylenchlorid aus und engt das Filtrat ein. Man erhält 730 mg des Laktons als amorphen Feststoff.

NMR ($CDCl_3$): δ = 0,68 ppm (S, 3H, H-18); 2,96 (s, 6H, $N(CH_3)_2$); 4,42 (m, 1H, H-11); 5,81 (m, 1H, H-4); 6,69 u. 7,02 (AA'BB'-, je 2H, arom. H).

Beispiel 2

3-β- [4- (2-Dimethylaminoethoxy)- phenyl]-17β-hydroxy -18- methyl -3- oxo-4,9(10)-estradien-17α-yl- propionsäurelakton

Eine Lösung von 3,4 g 11β-[4-(2-Dimethylaminoethoxy)-phenyl]- 3,3-(2,2-dimethylpropan-1,3-dioxy) -18- methyl-17α-[3-tetrahydropyran -2- yloxy)-propyl]-9(10)-estren-5α, 17β-diol in 120 ml 90%igen wässrigen Ethanol wird nach Zusatz von 1,75 g p-Toluolsulfonsäure 15 Minuten unter Rückfluss erhitzt. Nach dem Abkühlen giesst man in Eiswasser, stellt durch Zugabe von $NH_3$-Lösung einen pH-Wert von 10,5 ein und extrahiert mit Methylenchlorid. Chromatographie über Kieselgel mit Hexan/Essigester ergibt 1,86 g 11β[4-(2-Dimethylaminoethoxy)-phenyl]- 17β- hydroxy- 17α- (3-hydroxypropyl) -18- methyl-4,9(10)-estradien -3- on als festen Schaum.

$^1H$–NMR ($CDCl_3$): δ = 0,32 ppm (t, 3H, $CH_2$–$\underline{CH_3}$); 2,32 (s, 6H, $N(CH_3)_2$); 2,70 (t, 2H, $CH_2$–$\underline{CH_2}$–N); 4,01 (t, 2H, $CH_2$–$\underline{CH_2}$–O); 4,36

(m, 1H, H-11); 5,74 (m, 1H, H-4). UV (Methanol): $\lambda_{max}$282 (10 400); 289 (13 600); 306 nm (ε =18 720).

Oxidation von 1,6 g obigen Produkts unter den Bedingungen des Beispiels 1 (Absatz 2) ergibt 1,3 g des Laktons als gelbliches, amorphes Glas. IR ($CHCl_3$): 1730 $cm^{-1}$ (Laktoncarbonyl); 1685 (C =O).

Beispiel 3

3-[17β- Hydroxy-11β-(4-methoxyphenyl) -3-oxo- 4,9(10)-estradien- 17α- yl]-propionsäurelakton

Eine Lösung von 7,05 g 3,3-(2,2-Dimethylpropan- 1,3-dioxy)- 11β- (4-methoxyphenyl)-17α- [3-tetrahydropyran -2- yloxy)- propyl]-9(10)-estren- 5α, 17α-diol wird in 50 ml 70%ige wässrige Essigsäure 2 Stunden bei 60 °C gerührt. Nach dem Abkühlen giesst man in Wasser und extrahiert die saure, wässrige Lösung mit Methylenchlorid. Chromatographie des so erhaltenen Rohprodukts über Kieselgel mit Hexan/Essigester ergibt 3,8 g 17β-Hydroxy-17α-(3-hydroxypropyl)- 11β- (4-methoxy- phenyl)- 4,9(10)- estradien -3- on als gelblichen Schaum.

$^1$H–NMR ($CDCl_3$): δ = 0,56 ppm (s, 3H, H-18); 3,76 (s, 3H, $OCH_3$); 4,33 (m, 1H, H-11); 5,71 (m, 1H, H-4); 6,74 und 7,03 (AA'BB', je 2H, aromat. H).

Eine Lösung von 2,5 g des obig erhaltenen Produkts in 150 ml Methylenchlorid wird bei Raumtemperatur portionsweise mit 3,3 g Pyridiniumchlorochromat versetzt und anschliessend 2 Stunden bei 25 °C gerührt. Danach filtriert man über Celite, wäscht das Filtrat zunächst mit $NaHCO_3$-Lösung, dann mit gesättigter $NH_4Cl$-Lösung, trocknet über Natriumsulfat/Aktivkohle und engt ein. Säulenchromatographie über Kieselgel mit Hexan/Essigester liefert 1,45 g des Laktons.

$^1$H–NMR ($CDCl_3$): δ = 0,62 ppm (s, 3H, H-18); 3,77 (s, 3H, $OCH_3$); 4,40 (m, 1H, H-11); 5,77 (s, 1H, H-4); 6,91 (AA'BB', 4H, aromat. H).

Beispiel 4

11β- (4-Dimethylaminophenyl)- 17β- hydroxy- 17α- (3-hydroxypropyl)- 16β- methyl-4,9(10)-estradien -3- on

Die Spaltung von 3,42 g 11β-(4-Dimethylaminophenyl)- 3,3- (2,2-dimethyl- propan- 1,3-dioxy)- 16β-methyl- 17α- [3-(tetrahydropyran -2-yloxy)- propyl]- 9(10)-estren- 5α, 17β-diol mit 60 ml 70%iger wässriger Essigsäure unter den Bedingungen des Beispiels 1 ergibt 1,97 g 11β-(4-Dimethylaminophenyl)- 17β-hydroxy- 17α-(3-hydroxypropyl)- 16β- methyl- 4,9(10)-estradien -3- on als gelbliches Öl.

$^1$H–NMR ($CDCl_3$): δ = 0,51 ppm (s, 3H, H-18); 0,96 [d(J =7 Hz), 3H, 16β–$CH_3$]; 2,88 [s, 6H, $N(CH_3)_2$]; 3,63 (m, 2H, $CH_2OH$); 4,32 (m, 1H, H-11); 5,73 (s, 1H, H-4); 6,60 und 6,98 (AA'BB', je 2H aromat. H).

Beispiel 5

11β(4-Dimethylaminophenyl)- 17aβ-hydroxy-17aα- (3-hydroxypropyl)- D-homo- 4,9(10)-estradien -3- on

Aus 1,43 g 3,3-(2,2-Dimethyl-propan-1,3-dioxy)- 5α, 10α-oxido- D-homo- 9(11)-estren-17a-on erhält man nach Durchführung der Reaktionssequenz 1a–d (1d = Beispiel 1) ohne Reinigung der Zwischenstufen 630 mg 11β-(4-Dimethylaminophenyl- 17aβ-hydroxy-17aα-(3-hydroxy-propyl)- D-homo- 4,9(10)-estradien -3- on als gelblichen Schaum.

$^1$H–NMR ($CDCl_3$): δ = 0,58 ppm (s, 3H, H-18); 2,97 [s, 6H, $N(CH_3)_2$]; 4,36 (m, 1H, H-11); 5,74 (s, 1H, H-4); 6,63 und 6,69 (AA'BB', je 2H, aromat. H).

Beispiel 6

11β- (4-Dimethylaminophenyl)- 17β- hydroxy- 17α- (3-hydroxypropyl) -18- methyl-4,9(10)- estradien -3- on

Aus 4,32 g von unter 2 a) hergestelltem 3,3-(2,2-Dimethylpropan-1,3-dioxy) -18- methyl-5α, 10α- oxido- 17α- [3-(tetrahydropyran -2- yloxy)-prop -1- inyl]- 9(11)-estren- 17β-ol erhält man nach Umsetzung unter den Bedingungen von 1 b) – d) (1d = Beispiel 1) 2,25 g 11β-(4-Dimethylaminophenyl) -17β- hydroxy- 17α-(3-hydroxypropyl) -18- methyl-4,9(10)-estradien -3- on als hellgelbes Öl.

$^1$H–NMR ($CDCl_3$): δ =0,32 ppm [t(J=7 Hz), 3H, 13–$CH_2$–$\underline{CH_3}$]; 2,90 [s, 6H, $N(CH_3)_2$]; 3,68 (m, 2H, $CH_2\underline{OH}$); 4,31 (m, 1H, H-11); 5,72 (s, 1H, H-4); 6,63 und 7,03 (AA'BB', je 2H, aromat. H). UV (Methanol): $\lambda_{max}$260 nm (ε = 17 090); 307 (ε =19 500).

Beispiel 7

11β- [4-(2,5-Dimethylpyrrol -1- yl)- phenyl]-17β-hydroxy- 17α-(3-hydroxy- propyl)-4,9(10)-estradien -3- on

Eine Lösung von 0,98 g 3,3-(2,2-Dimethylpropan-1,3-dioxy)- 11β-[4-(2,5-dimethylpyrrol -1-yl)phenyl]-17α-[3(tetrahydropyran -2- yloxy)-propyl]- 9(10)-estren- 5α, 17β-diol und 100 mg p-Toluolsulfonsäure-Monohydrat in 30 ml Ethanol wird 2 Stunden bei Raumtemperatur gerührt. Danach giesst man auf ein Gemisch aus 10 ml konzentrierter Ammoniaklösung und 50 g Eis und extrahiert mit Essigester. Die Chromatographie an $Al_2O_3$ (neutral, III) mit Hexan/Essigester ergibt 0,36 g 11β-[4-(2,5-Dimethylpyrrol -1- yl)-phenyl]- 17β-hydroxy- 17α- (3-hydroxypropyl)-4,9(10)-estradien -3- on als zitronengelben festen Schaum.

Beispiel 8

17β-Hydroxy- 17α-(3-hydroxypropyl)-11β-(4-piperidinophenyl)-4,9(10)- estra-dien -3- on

Eine Lösung von 6,86 g 3,3- (2,2-Dimethylpropan-1,3-dioxy)- 11β- (4-piperidinophenyl)-17α-[3-(tetrahydropyran -2- yloxy)- propyl]-9(10)-estren-5α, 17β-diol in 150 ml 70% Essigsäure wird über Nacht bei Raumtemperatur und anschliessend 1 Stunde bei 50 °C gerührt. Nach dem Abkühlen giesst man auf ein Gemisch aus 30 ml konzentrierter Ammoniaklösung und 100 g

Eis und extrahiert mit Essigester. Die Chromatographie an Kieselgel ergibt 4,1 g 17β-Hydroxy-17α-(3-hydroxypropyl)- 11β- (4-piperidinophenyl)-4,9(10)-estradien -3- on als zitronengelben festen Schaum.

Beispiel 9

17β-Hydroxy-17α-(3-hydroxypropyl)-11β-[4-(pyrrolidin -1- yl)phenyl]- 4,9(10)- estra-dien-3- on

Eine Lösung von 6,3 g 3,3- (2,2-Dimethylpropan- 1,3-dioxy)- 11β- [4-(pyrrolidin -1- yl)phenyl]- 17α- [3-(tetrahydropyran -2- yloxy)propyl]-9(10)-estren-5α, 17β- diol in 150 ml 70% Essigsäure wird über Nacht bei Raumtemperatur gerührt. Anschliessend erwärmt man für eine Stunde auf 50 °C. Nach dem Abkühlen giesst man die Lösung auf ein Gemisch aus 30 ml konzentrierter Ammoniaklösung und 100 g Eis und extrahiert mit Essigester. Die Chromatographie an $SiO_2$ ergibt 3,5 g 17β- Hydroxy- 17α-(hydroxypropyl)- 11β- [4-(pyrrolidin -1- yl)phenyl]-4,9-(10)-estradien -3- on als zitronengelben festen Schaum.

Beispiel 10

Zu einer Suspension von 3,6 g 11β-(4-Dimethylaminophenyl)- 3,3- ethylendioxy- 5α-hydroxy- 9- estren -17- on und 3,3 g Trimethylsulfoniumjodid in 38 ml Dimethylformamid werden unter Argon bei Raumtemperatur 2,3 g Kalium-tert.-butylat innerhalb von 10 Minuten portionsweise gegeben. Nach 30 Minuten wird das Gemisch in Eis/Wasser eingerührt. Das ausgefallene Produkt wird abgesaugt, mit Wasser gewaschen, in Essigester gelöst und über Natriumsulfat getrocknet. Nach Chromatographie an Kieselgel mit Aceton/Hexan erhält man 3,1 g 11β-(4-Dimethylaminophenyl)- 3,3-ethylendioxy -9- estren [17(β-1')-spiro- 3']oxiran- 5α-ol.

Schmelzpunkt 128,0 °C (unter Zersetzung).

Beispiel 11

1,2 g 3,3-Ethylendioxy- 5α- hydroxy- 11β-(4-methoxyphenyl) -9- estren -17- on werden analog Beispiel 10 mit Trimethylsulfoniumjodid und Kalium-tert.-butylat in Dimethylformamid umgesetzt. Nach Chromatographie an Kieselgel mit Aceton/Hexan werden 860 mg 3,3- Ethylendioxy- 11β- (4-methoxyphenyl) -9- estren [17(β-1')-spiro- 3']oxiran- 5α-ol erhalten.

Schmelzpunkt 98 °C (unter Zersetzung).

Beispiel 12

3 g tert.-Butylmethylsulfoxid in 35 ml absolutem Tetrahydrofuran werden tropfenweise unter Argon bei 0 °C mit 15,6 ml einer 1,6 mol. n-Butyllithium-Lösung in Hexan versetzt. Anschliessend wird die Lösung von 4,6 g 11β-(4-Dimethylaminophenyl)- 3,3- ethylendioxy -9- estren [17(β-1')- spiro- 3']oxiran- 5α-ol in 30 ml Tetrahydrofuran zugetropft. Es wird bei Raumtemperatur 10 Stunden gerührt. Anschliessend wird das Gemisch in Eis/Wasser eingerührt und mit Essigester extrahiert. Die Lösung wird mit Wasser gewaschen und über Natriumsulfat getrocknet. Nach Chromatographieren an Kieselgel mit Aceton/Hexan werden 3,9 g 11β- (4-Dimethylaminophenyl)- 21- (1,1-dimethylethylsulfinyl)-3,3-ethylendioxy- 19- nor-17α-pregn -9- en-5α, 17β-diol als Diastereomerengemisch isoliert, das als fester Schaum anfällt.

Beispiel 13

3,6 g 3,3- Ethylendioxy- 11β- (4-methoxyphenyl) -9- estren- [17(β- 1')- spiro- 3']oxiran-5α-ol werden analog Beispiel 12 mit tert.-Butylmethylsulfoxid zu 21- (1,1-Dimethylethylsulfinyl)-3,3-ethylendioxy- 11β- (4-methoxyphenyl) -19-nor-17α- pregn -9- en-5α, 17β-diol als Diastereomerengemisch umgesetzt. Man erhält nach Chromatographieren an Kieselgel mit Aceton/Hexan 2,3 g eines festen Schaumes.

Beispiel 14

1,8 g 11β-(4-Dimethylaminophenyl) -21-(1,1- dimethylethylsulfinyl)- 3,3-ethylendioxy-19- nor-17α- pregn -9- en- 5α, 17β-diol (als Diastereomerengemisch) werden in 50 ml Eisessig und 20 ml Wasser 20 Stunden bei Raumtemperatur gerührt. Man erhält 1,1 g 11β- (4-Dimethylaminophenyl) -21- (1,1-dimethylethylsulfinyl)-17β-hydroxy -19- nor- 17α- pregna- 4,9-dien-3- on als Diastereomerengemisch, das als fester Schaum anfällt.

Beispiel 15

2,1 g 21- (1,1- Dimethylethylsulfinyl)- 3,3-ethylendioxy- 11β- (4-methoxyphenyl) -19-nor-17α- pregn -9- en- 5α, 17β-diol (als Diastereomerengemisch) werden analog Beispiel 14 mit wässrigem Eisessig zu 21-(1,1-Dimethylethylsulfinyl)- 17β-hydroxy- 11β- (4-methoxyphenyl) -19- nor- 17α-pregna- 4,9-dien -3- on (Diastereomerengemisch) umgesetzt. Es werden 1,3 g als schaumiges Produkt erhalten.

Beispiel 16

1,6 g 11β- (4-Dimethylaminophenyl) -21-(1,1- dimethylethylsulfinyl)-17β-hydroxy -19-nor- 17α- pregna- 4,9-dien -3- on (Diastereomerengemisch) werden in 30 ml Tetrahydrofuran und 10 ml Wasser bei Raumtemperatur mit 520 mg N-Chlorsuccinimid gerührt. Nach 2 Stunden wird das Gemisch mit Essigester verdünnt, mehrmals mit Wasser gewaschen und über Natriumsulfat getrocknet. Nach Chromatographieren des Rohproduktes an Kieselgel mit Aceton/Hexan werden 420 mg 11β-(4-Dimethylaminophenyl) -3- oxo-4,9-estradien [17(β-1')-spiro-5'] [1,2]-oxathiolan- 2'-oxid vom Schmelzpunkt 98 °C erhalten.

Beispiel 17

1,3 g 21- (1,1-Dimethylethylsulfinyl)- 17β-hydroxy- 11β- (4-methoxyphenyl) -19- nor-17α-pregna -4,9- dien -3- on (Diastereomerengemisch) werden analog Beispiel 16 mit N-Chlorsuccinimid umgesetzt. Man erhält nach Chromatographieren an Kieselgel mit Aceton/Hexan als Diastereomerengemisch 180 mg 11β-(4-Methoxyphenyl) -3- oxo-4,9-estradien [17(β- 1')-spiro- 5'] [1,2]-oxathiolan- 2'-oxid vom Schmelzpunkt 95 °C und 390 mg 11β-(3-Chlor- 4- methoxyphenyl) -3- oxo-4,9-estradien [17(β-1')-spiro-5'] [1,2]- oxathiolan- 2'-oxid als festen Schaum.

Beispiel 18

a) Es werden 1,5 g 11β-(4-Dimethylaminophenyl)-3,3- ethylendioxy -9- estren[17(β-1')-spiro-3']oxiran- 5α-ol mit 140 mg p-Toluolsulfonsäure und 5 ml flüssigem Methylamin vermischt und in einem Stahlgefäss 17 Stunden bei 130 °C Ölbadtemperatur erwärmt. Nach dem Abkühlen und Öffnen des Bombenrohres wird überschüssiges Methylamin nach Überführung in eine Kristallisationsschale abgedampft. Der Rückstand wird in Methylenchlorid aufgenommen, mit Wasser neutral gewaschen und nach dem Trocknen über Natriumsulfat eingeengt. Man erhält 1,7 g 11β-(4-Dimethylaminophenyl)-3,3-ethylen-dioxy-17α-methylaminomethyl -9- estren-5α, 17β-diol als Rohprodukt.

b) Eine Lösung von 1,6 g 11β-(4-Dimethylaminophenyl)- 3,3-ethylendioxy- 17α-methylaminomethyl -9- estren- 5α, 17β-diol und 800 mg Kalium-tert.-butylat in 10 ml Kohlensäuredimethylester wird 45 Minuten unter Rückfluss erhitzt. Nach dem Abkühlen wird mit Methylenchlorid verdünnt, mit Wasser neutral gewaschen, über Natriumsulfat getrocknet und im Vakuum zur Trockne eingeengt. Das Rohprodukt wird an 135 g Kieselgel mit einem Hexan-Aceton-Gradienten (0–60% Aceton) gereinigt. Man isoliert 650 mg 11β- (4-Dimethylaminophenyl)-3,3- ethylendioxy- 3'-methyl- 4,9- estradien [17(β- 1')- spiro- 5'] oxazolidin- 2'-on vom Schmelzpunkt 228–229 °C.

$[\alpha]_D^{25} = +101.5\,°C.$

Beispiel 19

490 mg 11β- (4-Dimethylaminophenyl)-3,3-ethylendioxy-3'-methyl-4,9-estradien [17(β-1')-spiro- 5']oxazolidin- 2'-on werden in 13,7 ml Eisessig und 5,3 ml Wasser 20 Stunden bei Raumtemperatur gerührt. Man gibt auf Eis/Wasser, stellt mit Kaliumhydroxyd (fest) schwach alkalisch, filtriert ab und nimmt den Rückstand in Methylenchlorid auf. Die organische Lösung wird nach dem Neutralwaschen und Trocknen eingeengt. Man isoliert nach dem Chromatographieren an Kieselgel mit Hexan/Aceton 430 mg 11β- (4-Dimethylaminophenyl)- 3'-methyl- 4,9-estradien [17 (β-1')- spiro- 5']-oxazolidin- 2',3- dion als schaumiges Produkt.

Beispiel 20

Ein Gemisch aus 2,8 g 3,3-Ethylendioxy-11β-(4-methoxyphenyl) -9- estren [17(β-1')- spiro-3']oxiran- 5α-ol, 8,4 g N-Methylurethan und 0,7 g Kalium-tert.-butylat werden in 20 ml Hexamethylphosphorsäuretriamid unter Argon 4 Stunden bei 130 °C gerührt. Man gibt anschliessend auf Eis/Wasser und extrahiert das abgeschiedene ölige Produkt mit Methylenchlorid. Die organischen Extrakte werden mit Wasser gewaschen, getrocknet und eingedampft. Man isoliert 710 mg 3,3- Ethylendioxy- 5α-hydroxy-11β-(4-methoxyphenyl)- 3'-methyl -9- estren[17(β-1')-spiro-5']oxazolidin-2'-on als festen Schaum.

Beispiel 21

600 mg des in Beispiel 20 erhaltenen schaumigen Produkts werden unter den Bedingungen des Beispiels 19 mit 70%ig wässriger Essigsäure umgesetzt, aufgearbeitet und gereinigt. Man erhält 260 mg 11β- (4-Methoxyphenyl)- 3'-methyl-4,9-estradien [17(β-1')- spiro-5']- oxazolidin-2', 3-dion als amorphen Feststoff.

Beispiel 22

Es werden 10,5 g 3,3-Ethylendioxy-11β-(4-methoxyphenyl) -9- estren [17(β-1')-spiro-3'] oxiran-5α-ol mit 1,05 g p-Toluolsulfonsäure und 35 ml flüssigem Methylamin vermischt und in einem Stahlgefäss 16 Stunden auf 130 °C Ölbadtemperatur erwärmt. Nach dem Abkühlen und Öffnen des Bombenrohres wird überschüssiges Methylamin abgedampft und der Rückstand auf ein Filter gebracht. Man wäscht mit Wasser neutral, nimmt das Rohprodukt in Essigester auf, filtriert ab und versetzt das auf 1/4 seines Volumens eingeengte Filtrat mit Hexan. Es fallen 3,8 g 3,3-Ethylendioxy-11 β-(4-methoxyphenyl)-17α-methylaminomethyl -9- estren-5α, 17β-diol als amorpher Stoff an, der in 30 ml Triethylamin gelöst wird. Man gibt bei 5 °C 3 ml Thionylchlorid tropfenweise zu, rührt 30 Minuten und gibt das Reaktionsgemisch in Eis/Wasser. Es wird mit Essigester extrahiert, mit Wasser gewaschen und über Natriumsulfat getrocknet. Das so erhaltene Rohprodukt wird in 40 ml 70%iger Essigsäure 3 Stunden bei 60 °C gerührt und anschliessend wie in Beispiel 19 beschrieben aufgearbeitet. Man isoliert 900 mg 11β-(4-Methoxyphenyl)-3'-methyl -3- oxo- 4,9- estradien [17(β-1')- spiro-5'] [1,2,3]-oxathiazolidin-2'-oxid (als Diastereomerengemisch) als schaumiges Produkt.

**Patentansprüche für die Vertragsstaaten BE, CH, DE, FR, GB, IT, LU, NL, SE**

1. 11β-Aryl-Estradiene der allgemeinen Formel I

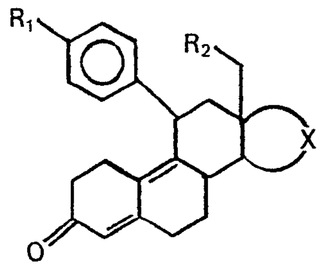

(I),

worin

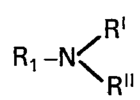

mit $R^I$ und $R^{II}$ in der Bedeutung

von jeweils Alkyl mit 1 bis 4 Kohlenstoffatomen oder $R^I$ und $R^{II}$ unter Einschluss von N in der Bedeutung eines 5- oder 6gliedrigen heterocyclischen Ringes, wobei im Ring ausser N noch ein weiteres Heteroatom N, O oder S enthalten sein kann und die entsprechenden N-Oxide,

$-SR^{III}$ mit $R^{III}$ in der Bedeutung von Methyl, Ethyl oder Phenyl und

$-OR^{IV}$ mit $R^{IV}$ in der Bedeutung von Methyl, Ethyl, Propyl, Methoxymethyl, Allyl oder β-Dimethylaminoethyl,

$R_2$ ein Wasserstoffatom, eine Methyl- oder Ethylgruppe und

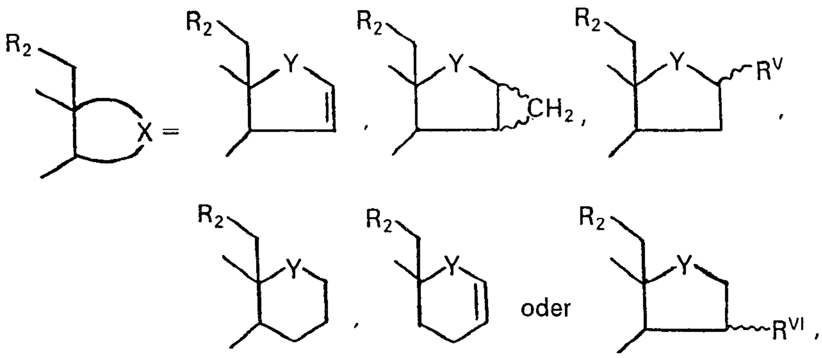

oder

wobei
die Wellenlinien  andeuten, dass der Substituent in α- oder β-Stellung und

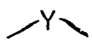 für 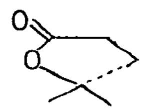 , 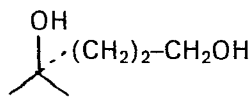 oder

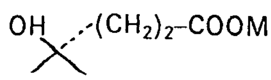 mit M = K, Na oder Li oder

für 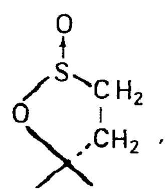 , 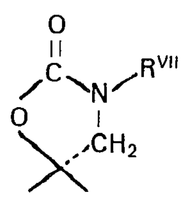 oder

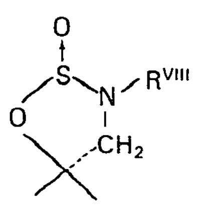

steht, und

$R^V$ und $R^{VI}$ und $R^{VII}$ und $R^{VIII}$ jeweils Alkyl mit 1 bis 4 Kohlenstoffatomen bedeuten.

2. 3-[11β-(4-Dimethylaminophenyl)-17β-hydroxy -3- oxo- 4,9(10)-estradien- 17α-yl]- propionsäure-lakton und propionsäure-Kaliumsalz.

3. 11β-(4-Dimethylaminophenyl)-17β-hydroxy- 17α- (3-hydroxypropyl)- 4,9(10)-estradien- 3- on.

4. 3- {11β- [4-(2-Dimethylaminoethoxy)-phenyl]- 17β-hydroxy -18- methyl -3- oxo- 4,9(10)-estradien- 17α-yl} propionsäurelakton und- propionsäure-Kaliumsalz.

5. 11β- [4-(2-Dimethylaminoethoxy)-phenyl]-17β-hydroxy- 17α- (3-hydroxypropyl) -18- methyl- 4,9(10)-estradien -3- on.

6. 3- [17β- Hydroxy- 11β-(4-methoxyphenyl)- 3- oxo- 4,9(10)-estradien- 17α-yl]-propionsäure-lakton und propionsäure-Natriumsalz.

7. 17β-Hydroxy- 17α- (3-hydroxypropyl)- 11β-(4- methoxyphenyl)- 4,9(10)-estradien -3- on.

8. 11β- (4-Dimethylaminophenyl)- 17β-hydroxy- 17α- (3-hydroxypropyl)- 16β- methyl- 4,9(10)-estradien -3- on.

9. 11β- (4-Dimethylaminophenyl)- 17β-hydroxy- 17α- (3-hydroxypropyl) -18- methyl- 4,9(10)-estradien -3- on.

10. 11β- (4-Dimethylaminophenyl)- 17aβ- hydroxy- 17aα- (3-hydroxypropyl)- D-homo- 4,9(10)- estradien -3- on.

11. 11β- [4-(2,5- Dimethylpyrol -1- yl)-phenyl]- 17β-hydroxy- 17α- (3-hydroxypropyl)- -4,9(10)- estradien -3- on.

12. 17β- Hydroxy- 17α- (3-hydroxypropyl)- 11β- (4-piperidinophenyl)- 4,9(10)-estradien- 3-on.

13. 17β- Hydroxy- 17α-( hydroxypropyl)- 11β- [4- (pyrrolidin -1- yl)phenyl]- 4,9(10)- estradien- 3- on.

14. 11β- (4-Dimethylaminophenyl) -3- oxo- 4,9- estradien [17(β- 1')-spiro- 5'] [1,2]-oxathiolan- 2'-oxid.

15. 11β- (4-Methoxyphenyl) -3- oxo- 4,9-estradien [17(β- 1')-spiro- 5'] [1,2]- oxathiolan-2'-oxid.

16. 11β- (4-Dimethylaminophenyl)- 3'-methyl-4,9-estradien [17(β- 1')-spiro- 5']-oxazolidin-2', 3-dion.

17. 11β- (4-Methoxyphenyl)- 3'-methyl-4,9-estradien [17(β- 1')-spiro- 5']-oxazolidin-2', 3-dion.

18. 11β- (4-Methoxyphenyl) -3'-methyl -3-oxo- 4,9-estradien [17(β- 1')-spiro- 5'] [1,2,3]-oxathiazolidin- 2'-oxid.

19. Pharmazeutische Präparate, gekennzeichnet durch den Gehalt an Verbindungen gemäss Anspruch 1–18.

20. Verfahren zur Herstellung von 11β-Aryl-Estradienen der allgemeinen Formel I

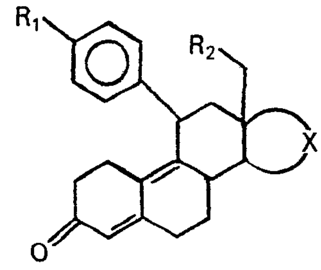

(I),

worin

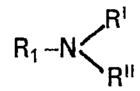 mit $R^I$ und $R^{II}$ in der Bedeutung von jeweils Alkyl mit 1 bis 4 Kohlenstoffatomen oder $R^I$ und $R^{II}$ unter Einschluss von N in der Bedeutung eines 5- oder 6gliedrigen heterocyclischen Ringes, wobei im Ring ausser N noch ein weiteres Heteroatom N, O oder S enthalten sein kann und die entsprechenden N-Oxide,

$-SR^{III}$ mit $R^{III}$ in der Bedeutung von Methyl, Ethyl oder Phenyl und

$-OR^{IV}$ mit $R^{IV}$ in der Bedeutung von Methyl, Ethyl, Propyl, Methoxymethyl, Allyl oder β-Dimethylaminoethyl,

$R_2$ ein Wasserstoffatom, eine Methyl- oder Ethylgruppe und

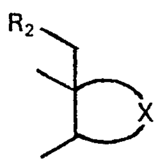 = 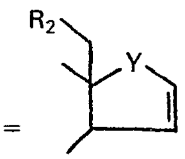, 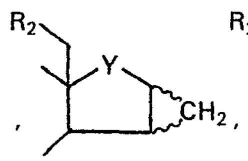, 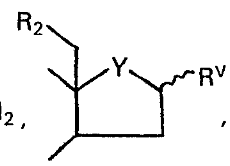,

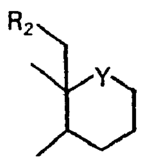, 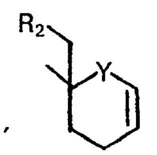 oder 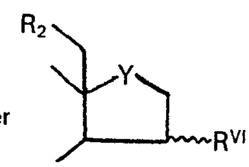,

wobei
die Wellenlinien andeuten, dass der Substituent in α- oder β-Stellung und

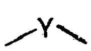 für 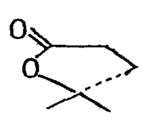, 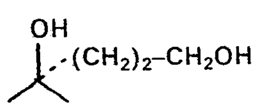 oder

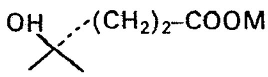 mit M = K, Na oder Li oder

für 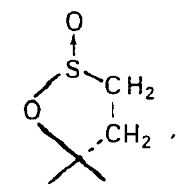 , 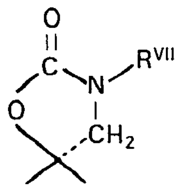 oder

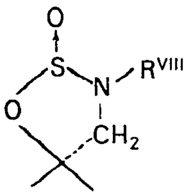

steht, und

$R^V$ und $R^{VI}$ und $R^{VII}$ und $R^{VIII}$ jeweils Alkyl mit 1 bis 4 Kohlenstoffatomen bedeuten, dadurch gekennzeichnet, dass man eine Verbindung der allgemeinen Formel II

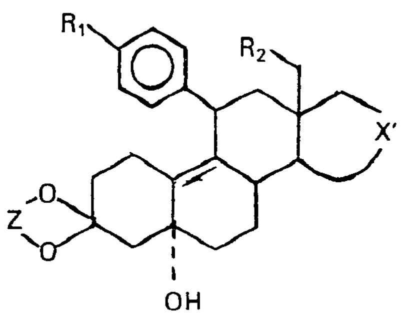

(II),

worin

$R_1$ und $R_2$ die in Formel I angegebene Bedeutung haben,

Z eine Ethylen- oder 2,2-Dimethylpropylengruppe darstellt und, falls

X' wie X in Formel I mit Y in der Bedeutung von

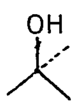 $(CH_2)_2$–$CH_2OR$, wobei R ein Wasserstoffatom oder eine leicht abspaltbare Gruppe darstellt, zur Herstellung von Verbindungen der Formel I mit

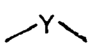 = 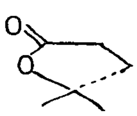 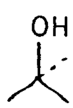 $(CH_2)_2$–$CH_2OH$

oder 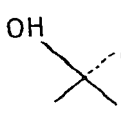 $(CH_2)_2$–COOM

mit M = K, Na oder Li

mit einer verdünnten Säure oder einem sauren Ionenaustauscher bei Temperaturen von 0–100 °C umsetzt und gegebenenfalls die so erhaltene 17α-(3-Hydroxypropyl)-Verbindung der allgemeinen Formel I in an sich bekannter Weise zum 17α-Propionsäurelakton oxydiert und gegebenenfalls den Laktonring mit Alkali zum entsprechenden 17α-Propionsäure-Alkalisalz öffnet,

und, falls

X' wie X in Formel I mit Y in der Bedeutung von

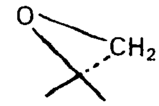

a) zur Herstellung von Verbindungen der Formel I

mit 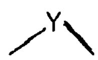 = 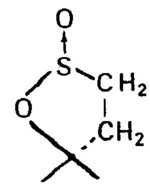

mit tert.-Butylmethylsulfoxid und Alkyllithium in Tetrahydrofuran, dann mit verdünnter Säure und anschliessend mit N-Chlor- oder N-Bromsuccinimid behandelt,

b) zur Herstellung von Verbindungen der Formel I

mit 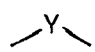 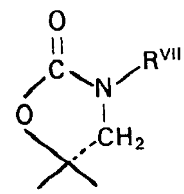

mit Alkylamin in Gegenwart einer Säure, dann mit Kohlensäuredialkylester in Gegenwart einer Base und anschliessend mit verdünnter Säure oder mit N-Alkylurethan und Kalium-tert.-butylat in Hexamethylphosphorsäuretriamid und dann mit verdünnter Säure umsetzt und

c) zur Herstellung von Verbindungen der Formel I

mit 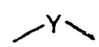 = 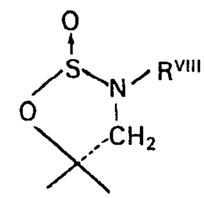

mit Alkylamin in Gegenwart von Säure, dann mit Thionylchlorid in Triethylamin und anschliessend mit verdünnter Säure behandelt.

**Patentanspruch für den Vertragsstaat AT**

Verfahren zur Herstellung von 11β-Aryl-Estradienen der allgemeinen Formel I

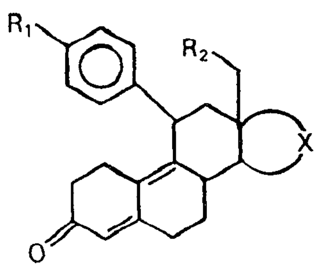

(I),

worin

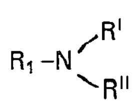 mit $R^I$ und $R^{II}$ in der Bedeutung

von jeweils Alkyl mit 1 bis 4 Kohlenstoffatomen oder $R^I$ und $R^{II}$ unter Einschluss von N in der Bedeutung eines 5- oder 6gliedrigen heterocyclischen Ringes, wobei im Ring ausser N noch ein weiteres Heteroatom N, O oder S enthalten sein kann und die entsprechenden N-Oxide,

$-SR^{III}$ mit $R^{III}$ in der Bedeutung von Methyl, Ethyl oder Phenyl und

$-OR^{IV}$ mit $R^{IV}$ in der Bedeutung von Methyl, Ethyl, Propyl, Methoxymethyl, Allyl oder β-Dimethylaminoethyl,

$R_2$ ein Wasserstoffatom, eine Methyl- oder Ethylgruppe und

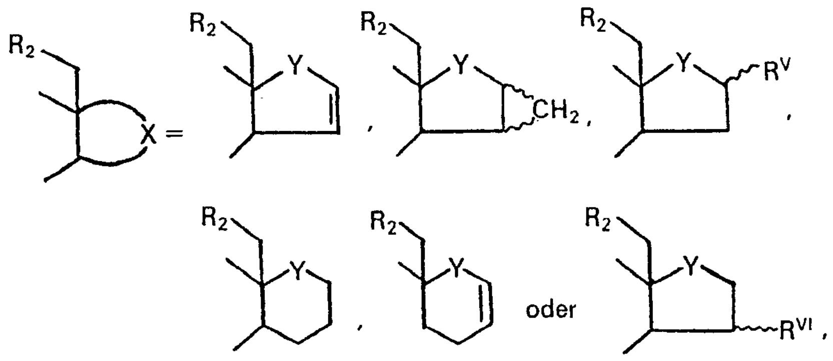

wobei die Wellenlinien andeuten, dass der Substituent in α- oder β-Stellung und

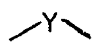 für 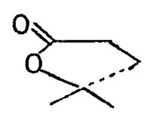, 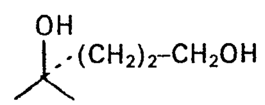 oder

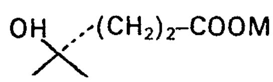 mit M = K, Na oder Li oder

für 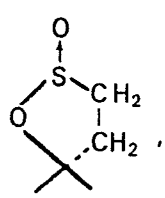, 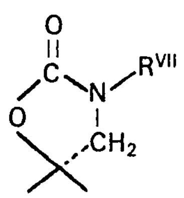 oder

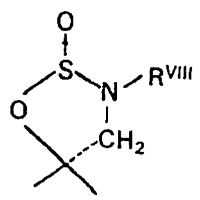

steht, und

$R^V$ und $R^{VI}$ und $R^{VII}$ und $R^{VIII}$ jeweils Alkyl mit 1 bis 4 Kohlenstoffatomen bedeuten, dadurch gekennzeichnet, dass man eine Verbindung der allgemeinen Formel II

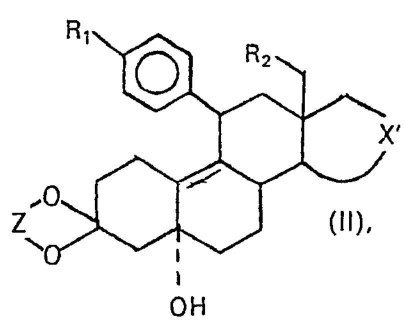

(II),

(II),

worin

$R_1$ und $R_2$ die in Formel I angegebene Bedeutung haben,

Z eine Ethylen- oder 2,2-Dimethylpropylengruppe darstellt und, falls

X' wie X in Formel I mit Y in der Bedeutung von

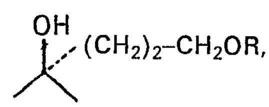, wobei R ein Wasserstoffatom oder eine leicht abspaltbare Gruppe darstellt, zur Herstellung von Verbindungen der Formel I mit

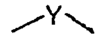 = 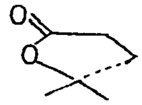 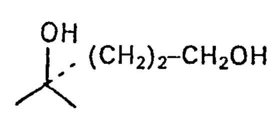

oder 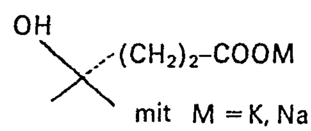

mit M = K, Na oder Li

mit einer verdünnten Säure oder einem sauren Ionenaustauscher bei Temperaturen von 0–100 °C umsetzt und gegebenenfalls die so erhaltene 17α-(3-Hydroxypropyl)-Verbindung der allgemeinen Formel I in an sich bekannter Weise zum 17α-Propionsäurelakton oxydiert und gegebenenfalls den

Laktonring mit Alkali zum entsprechenden 17α-Propionsäure-Alkalisalz öffnet,
und, falls

X' wie X in Formel I mit Y in der Bedeutung von

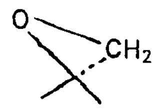

a) zur Herstellung von Verbindungen der Formel I

mit 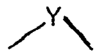 = 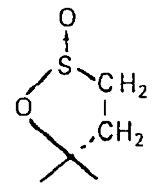

mit tert.-Butylmethylsulfoxid und Alkyllithium in Tetrahydrofuran, dann mit verdünnter Säure und anschliessend mit N-Chlor- oder N-Bromsuccinimid behandelt,

b) zur Herstellung von Verbindungen der Formel I

mit 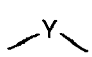 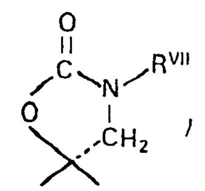 /

mit Alkylamin in Gegenwart einer Säure, dann mit Kohlensäuredialkylester in Gegenwart einer Base und anschliessend mit verdünnter Säure oder
mit N-Alkylurethan und Kalium-tert.-butylat in Hexamethylphosphorsäuretriamid und dann mit verdünnter Säure umsetzt und

c) zur Herstellung von Verbindungen der Formel I

mit 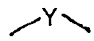 = 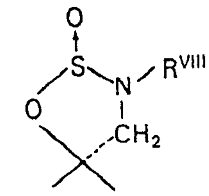

mit Alkylamin in Gegenwart von Säure, dann mit Thionylchlorid in Triethylamin und anschliessend mit verdünnter Säure behandelt.

**Revendications pour les états contractants BE, CH, DE, FR, GB, IT, LU, NL, SE**

1. Les nouveaux 11β-arylestradiènes de la formule générale I

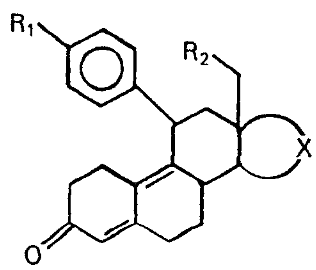 (I),

où

$R_1$ signifie 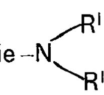 avec $R^I$ et $R^{II}$ chaque fois avec la signification de alcoyle avec 1 à 4 atomes de carbone ou $R^I$ et $R^{II}$ avec inclusion de N avec la signification d'un cycle hétérocyclique pentagonal ou hexagonal, le cycle pouvant contenir, outre N, un autre hétéroatome N, O ou S et les oxydes N correspondants,

$-SR^{III}$ avec $R^{III}$ avec la signification de méthyle, éthyle ou phényle et

$-OR^{IV}$ avec $R^{IV}$ avec la signification de méthyle, éthyle, propyle, méthoxyméthyle, allyle ou β-diméthylaminoéthyle.

$R_2$ signifie un atome d'hydrogène, un groupe méthyle ou éthyle et

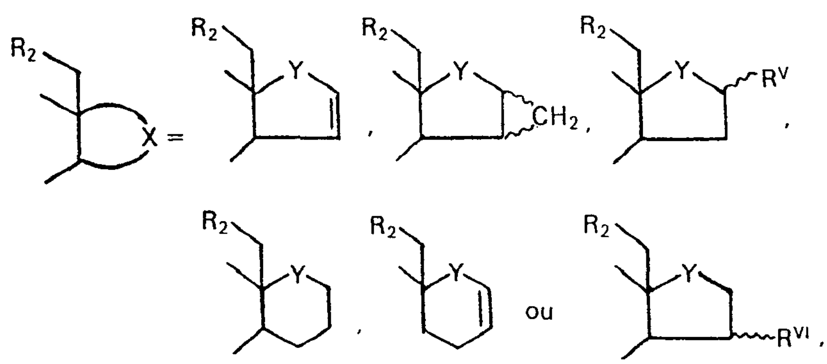

où
les lignes ondulées ~~~~ signifient que le substituant se trouve en position α ou β et

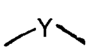 signifie 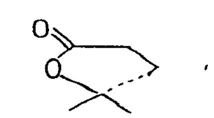 , 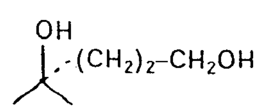 ou

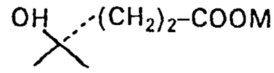 avec M = K, Na ou Li

ou 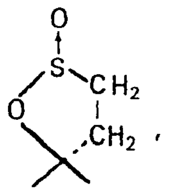 , 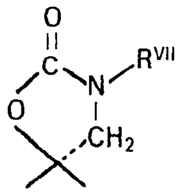 ou

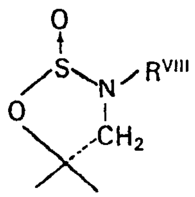

et

$R^V$ et $R^{VI}$ et $R^{VII}$ et $R^{VIII}$ signifient chaque fois un alcoyle avec 1 à 4 atomes de carbone.

2. Lactone et sel de potassium de l'acide 3-[11β- 4- diméthylamino- phényle)- 17β-hydroxy -3- oxo-4,9(10)-estradiène-17α-yl]-propionique.

3. 11β- (4-diméthylaminophényle)- 17β-hydroxy- 17α-(3-hydroxypropyle)- 4,9(10)- estradién -3- one.

4. Lactone et sel de potassium de l'acide 3-11β[4- (2-diméthylamino- éthoxy)- phényle]- 17β-hydroxy -18- méthyle -3- oxo-4,9(10)-estradién-17α-yl-propionique.

5. 11β- [4- (2-diméthylaminoéthoxy)- phényle]- 17β-hydroxy- 17α-(3-hydroxypropyle) -18-méthyle-4,9(10)-estradién -3- one.

6. Lactone et sel de sodium de l'acide 3-[17β-hydroxy-11β-(4-méthoxyphényle) -3- oxo-4,9(10)-estradién-17α-yl]-propionique.

7. 17β-hydroxy-17α-(3-hydroxypropyle)-11β-(4-méthoxyphényle)-4,9(10)-estradién -3- one.

8. 11β- (4-diméthylaminophényle)- 17β-hydroxy- 17α- (3-hydroxypropyle)- 16β-méthyle-4,9 (10)-estradién -3- one.

9. 11β-(4-diméthylaminophényle)-17β-hydroxy- 17α- (3-hydroxypropyle) -18- méthyle-4,9(10)-estradién -3- one.

10. 11β-(4-diméthylaminophényle)-17aβ-hydroxy-17aα-(3-hydroxypropyle)-D-homo-4,9(10)-estradién -3- one.

11. 11β-[4-(2,5-diméthylpyrrol -1- yl)-phényle]-17β-hydroxy-17α-(3-hydroxypropyle)-4,9(10)-estradién -3- one.

12. 17β- hydroxy- 17α-(3-hydroxypropyle)-11β- (4-pipéridinophényle)- 4,9(10)-estradién -3- one.

13. 17β-hydroxy-17α-(hydroxypropyle)-11β-[4-(pyrrolidin -1- yl)-phényle]- 4,9(10)-estradién -3- one.

14. 11β-(4-diméthylaminophényle) -3- oxo-4,9-estradiène-[17(β-1')-spiro-5'][1,2]-oxathiolane-2'-oxyde.

15. 11β-(4-méthoxyphényle) -3- oxo-4,9-estradiène-[17(β-1')-spiro-5'] [1,2]-oxathiolane-2'-oxyde.

16. 11β-(4-diméthylaminophényle)-3'-méthyle-4,9-estradiène-[17(β-1')-spiro-5']-oxazolidine-2', 3-dione.

17. 11β-(4-méthoxyphényle)-3'-méthyle-4,9-estradiène- [17(β-1')- spiro-5']- oxazolidine-2', 3-dione.

18. 11β-(4-méthoxyphényle)-3'-méthyle -3-oxo-4,9-estradiène-[17(β-1')-spiro-5'] [1,2,3]-oxathiazolidine-2'-oxyde.

19. Préparations pharmaceutiques caractérisées par un contenu en composés conformes aux revendications 1 à 18.

20. Méthodes pour la fabrication de 11β-aryl-estradiènes de la formule générale I

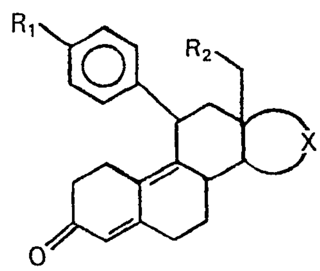 (I),

où

$R_1$ signifie 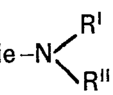 avec $R^I$ et $R^{II}$ chaque fois avec la signification de alcoyle avec 1 à 4 atomes de carbone ou $R^I$ et $R^{II}$ avec inclusion de N avec la signification d'un cycle hétérocyclique pentagonal ou hexagonal, le cycle pouvant contenir, outre N, un autre hétéroatome N, O ou S et les oxydes N correspondants,

$-SR^{III}$ avec $R^{III}$ avec la signification de méthyle, éthyle ou phényle et

$-OR^{IV}$ avec $R^{IV}$ avec la signification de méthyle, éthyle, propyle, méthoxyméthyle, allyle ou β-diméthylaminoéthyle.

$R_2$ signifie un atome d'hydrogène, un groupe méthyle ou éthyle et

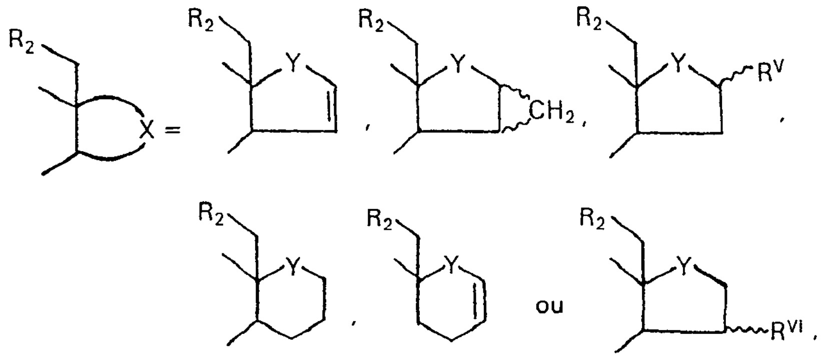

où

les lignes ondulées signifient que le substituant se trouve en position α ou β et

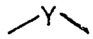 signifie 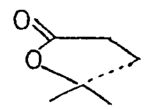 , 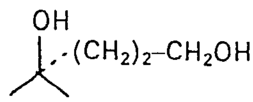 ou

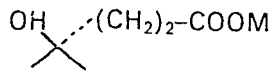 avec M = K, Na ou Li

ou 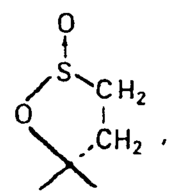 , 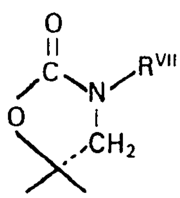 ou

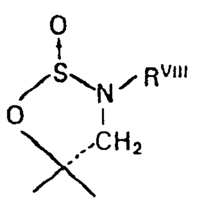

et

$R^V$ et $R^{VI}$ et $R^{VII}$ et $R^{VIII}$ signifient chaque fois un alcoyle avec 1 à 4 atomes de carbone, caractérisé en ce que l'on soumet un composé de la formule générale II

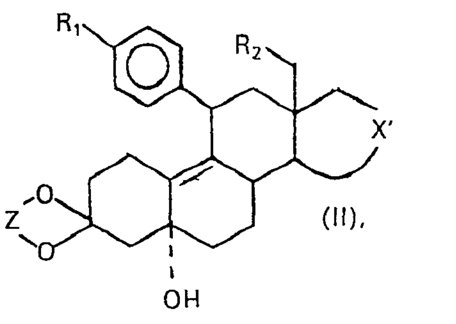

(II),

(II),

où

$R_1$ et $R_2$ ont la signification indiquée dans la formule I,

Z représente un groupe éthylène ou 2,2-diméthylepropylène et, dans le cas où

X' a la même signification que X dans la formule I avec Y avec la signification de

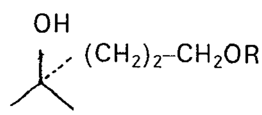

où R représente un atome d'hydrogène ou un groupe facilement séparable, pour la fabrication de composés suivant la formule I avec

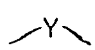 = 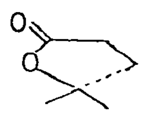 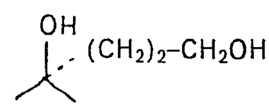

ou 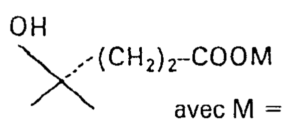

avec M = K, Na ou Li

d'un acide dilué ou d'un échangeur d'ions acide à des températures de 0 à 100 °C et le composé 17α-(hydroxypropyle)- suivant la formule générale I, ainsi obtenu, est, le cas échéant, oxydé d'une manière connue en lactone d'acide 17α-propionique et le cycle de lactone est, le cas échéant, rompu avec de l'alcali pour former le sel alcalin de l'acide 17α-propionique, et, dans le cas où

X' a la même signification que X dans la formule I avec Y avec la signification de

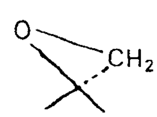

a) pour la fabrication de composés suivant la formule I

avec 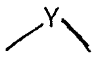 = 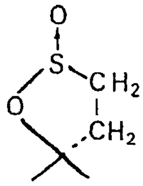

est traité avec le sulfoxyde butylméthylique tertiaire et l'alcoyllithium dans le tétrahydrofurane, ensuite avec de l'acide dilué et enfin avec N-chloro- ou N-bromosuccinimide,

b) pour la fabrication de composés suivant la formule I

avec 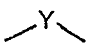 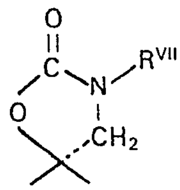

est fait réagir tout abord avec l'alcoylamine en présence d'acide, en suite avec le dialcoylester de l'acide carbonique en présence d'une base et enfin avec de l'acide dilué
ou avec N-alcoyluréthane et butylate tertiaire de potassium dans le triamide d'acide phosphorique hexaméthylique et ensuite avec de l'acide dilué et

c) pour la fabrication de composés suivant la formule I

avec 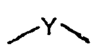 = 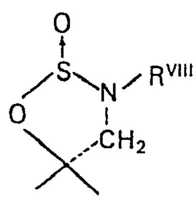

est traité avec de l'alcoylamine en présence d'acide, ensuite avec du chlorure de thionyle en présence de la triéthylamine et enfin avec de l'acide dilué.

**Revendication pour l'état contractant: AT**

Méthodes pour la fabrication de 11β-arylestradiènes de la formule générale I

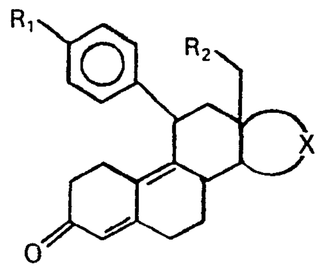

(I),

où

$R_1$ signifie 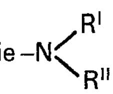 avec $R^I$ et $R^{II}$ chaque fois avec la signification de alcoyle avec 1 à 4 atomes de carbone ou $R^I$ et $R^{II}$ avec inclusion de N avec la signification d'un cycle hétérocyclique pentagonal ou hexagonal, le cycle pouvant contenir, outre N, un autre hétéroatome N, O ou S et les oxydes N correspondants,

$-SR^{III}$ avec $R^{III}$ avec la signification de méthyle, éthyle ou phényle et

$-OR^{IV}$ avec $R^{IV}$ avec la signification de méthyle, éthyle, propyle, méthoxyméthyle, allyle ou β-diméthylaminoéthyle.

$R_2$ signifie un atome d'hydrogène, un groupe méthyle ou éthyle et

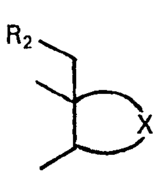 = 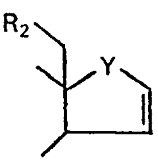 , 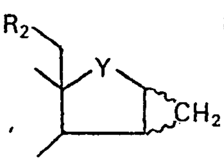 , 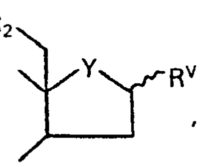 ,

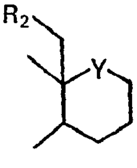 , 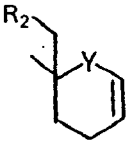 ou 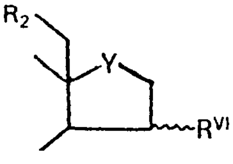 ,

où

les lignes ondulées ~~~ signifient que le substituant se trouve en position α ou β et

 signifie  ,  ou

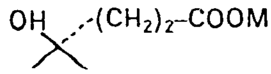 avec M = K, Na ou Li

ou 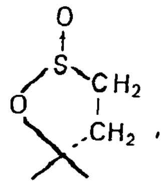 , 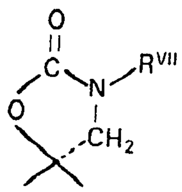 ou

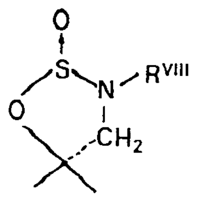

et

$R^V$ et $R^{VI}$ et $R^{VII}$ et $R^{VIII}$ signifient chaque fois un alcoyle avec 1 à 4 atomes de carbone, caractérisé en ce que l'on soumet un composé de la formule générale II

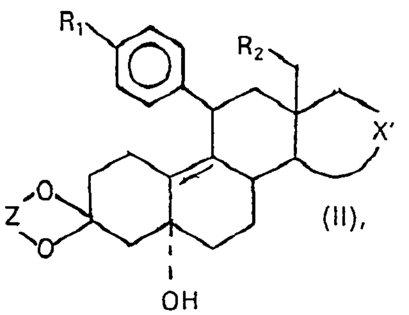

(II),

(II),

où

$R_1$ et $R_2$ ont la signification indiquée dans la formule I,

Z représente un groupe éthylène ou 2,2-diméthylepropylène et, dans le cas où

X' a la même signification que X dans la formule I avec Y avec la signification de

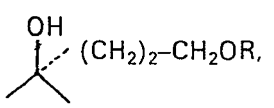

où R représente un atome d'hydrogène ou un groupe facilement séparable, pour la fabrication de composés suivant la formule I avec

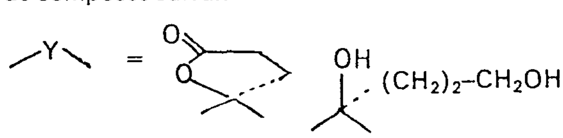

ou

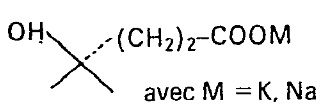

avec M = K, Na ou Li

d'un acide dilué ou d'un échangeur d'ions acide à des températures de 0 à 100 °C et le composé 17α-(hydroxypropyle)- suivant la formule générale I, ainsi obtenu, est, le cas échéant, oxydé d'une manière connue en lactone d'acide 17α-propionique et le noyau de lactone est, le cas échéant, rompu avec de l'alcali pour former le sel alcalin de l'acide 17α-propionique, et, dans le cas où

X' a la même signification que X dans la formule I avec Y avec la signification de

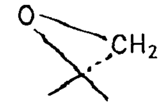

a) pour la fabrication de composés suivant la formule I

avec 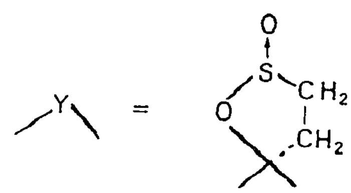

est traité avec le sulfoxyde butylméthylique tertiaire et l'alcoyllithium dans le tétrahydrofurane, ensuite avec de l'acide dilué et enfin avec N-chloro- ou N-bromosuccinimide,

b) pour la fabrication de composés suivant la formule I

avec 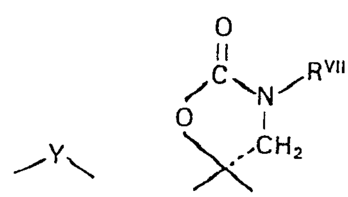

est fait réagir tout abord avec l'alcoylamine en présence d'acide, en suite avec le dialcoylester de l'acide carbonique en présence d'une base et enfin avec de l'acide dilué

ou avec N-alcoyluréthane et butylate tertiaire de potassium dans le triamide d'acide phosphorique hexaméthylique et ensuite avec de l'acide dilué

et

c) pour la fabrication de composés suivant la formule I

avec 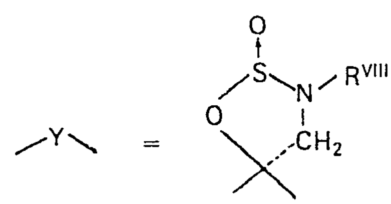

est traité avec de l'alcoylamine en présence d'acide, ensuite avec du chlorure de thionyle en présence de la triéthylamine et enfin avec de l'acide dilué.

**Patent claims for the contracting states: BE, CH, DE, FR, GB, IT, LU, NL, SE.**

1. 11β-Aryl-estradienes of the general formula I

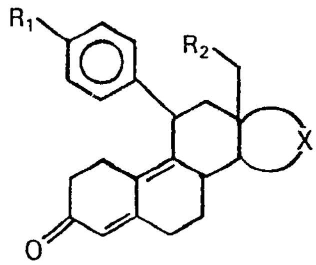

(I),

wherein

$R_1$ is 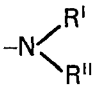 with $R^I$ and $R^{II}$ in each case being alkyl of 1 to 4 carbon atoms or $R^I$ and $R^{II}$ together with the N representing a 5- or 6- membered heterocyclic ring which besides the N can contain a further heteroatom N, O or S and the corresponding N-oxides,

$-SR^{III}$ with $R^{III}$ being methyl, ethyl or phenyl and

$-OR^{IV}$ with $R^{IV}$ being methyl, ethyl, propyl, methoxymethyl, allyl or β-dimethylaminoethyl,

$R^2$ is a hydrogen atom, a methyl or ethyl group and

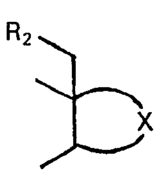 = 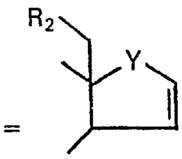 , 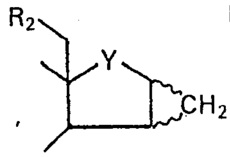 , 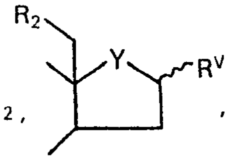 ,

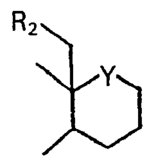 , 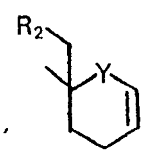 or 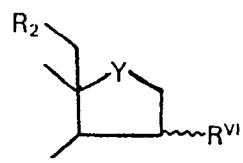 ,

in which the wavy line ∿∿ indicates that the substituent is in the α- or β-position and –Y– represents

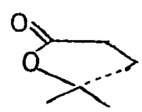 , 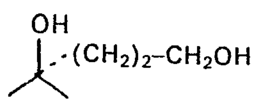 or 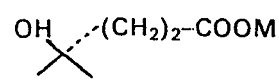

with M = K, Na or Li or

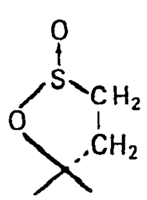 , 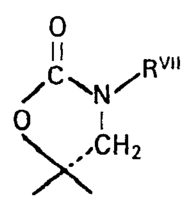 or 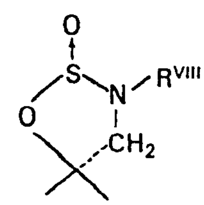

$R^V$ and $R^{VI}$ and $R^{VII}$ and $R^{VIII}$ in each case are alkyl of 1 to 4 carbon atoms.

2. 3-[11β-(4-Dimethylaminophenyl)-17β-hydroxy -3- oxo-4,9(10)-estradien-17α-yl]-propionic acid lactone and propionic acid-potassium salt.

3. 11β-(4-Dimethylaminophenyl)-17β-hydroxy-17α-(3-hydroxypropyl)-4,9(10)-estradien-3- one.

4. 3-[11β-[4-(2-Dimethylaminoethoxy)phenyl]-17β-hydroxy -18- methyl -3- oxo-4,9(10)-estradien-17α-yl]-propionic acid lactone and propionic acid-potassium salt.

5. 11β-[4-(2-Dimethylaminoethoxy)phenyl]-17β-hydroxy-17α-(3-hydroxypropyl) -18- methyl-4,9(10)-estradien -3- one.

6. 3-[17β-Hydroxy-11β-(4-methoxyphenyl) -3- oxo-4,9(10)-estradien-17α-yl]-propionic acid lactone and propionic acid-sodium salt.

7. 17β-Hydroxy-17α-(3-hydroxypropyl)-11β-(4-methoxyphenyl)-4,9(10)-estradien -3- one.

8. 11β-(4-Dimethylaminophenyl)-17β-hydro-

xy-17α-(3-hydroxypropyl)-16β-methyl-4,9(10)-estradien -3- one.

9. 11β-(4-Dimethylaminophenyl)-17β-hydroxy-17α-(3-hydroxypropyl) -18- methyl-4,9(10)-estradien -3- one.

10. 11β-(4-Dimethylaminophenyl)-17aβ-hydroxy-17aα-(3-hydroxypropyl)-D-homo-4,9(10)-estradien -3- one.

11. 11β-[4-(2,5-Dimethylpyrrol -1- yl)phenyl]-17β-hydroxy-17α-(3-hydroxypropyl)-4,9(10)-estradien -3- one.

12. 17β-Hydroxy-17α-(3-hydroxypropyl)-11β-(4-piperidinophenyl)-4,9(10)-estradien -3- one.

13. 17β-Hydroxy-17α-(3-hydroxypropyl)-11β-[4-(pyrrolidin -1- yl)phenyl]-4,9(10)-estradien -3- one.

14. 11β-(4-Dimethylaminophenyl) -3- oxo-4,9-estradien-[17(β-1')-spiro-5'] [1,2]-oxathiolan-2'-oxide.

15. 11β-(4-Methoxyphenyl) -3- oxo-4,9-estradien [17(β-1')-spiro-5'] [1,2]oxathiolan-2'-oxide.

16. 11β-(4-Dimethylaminophenyl)-3'-methyl-4,9-estradien[17(β-1')-spiro-5']-oxazolidin-2',3-dione.

17. 11β-(4-Methoxyphenyl)-3'-methyl-4,9-estradien[17(β-1')-spiro-5']-oxazolidin-2'3-dione.

18. 11β- (4- Methoxyphenyl) -3'- methyl -3-oxo-4,9-estradien[17(β-1')-spiro-5'] [1,2,3]-oxathiazolidin- 2'-oxide.

19. Pharmaceutical compositions, characterised by a content of compounds according to claims 1–18.

20. Process for the preparation of 11β-estradienes of the general formula I

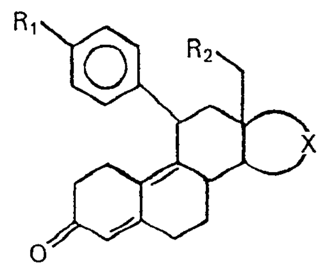

(I),

wherein

$R_1$ is 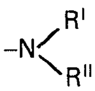 with $R^I$ and $R^{II}$ in each case being alkyl of 1 to 4 carbon atoms or $R^I$ and $R^{II}$ together with the N representing a 5- or 6-membered heterocyclic ring which besides the N can contain a further heteroatom N, O or S and the corresponding N-oxides,

$-SR^{III}$ with $R^{III}$ being methyl, ethyl or phenyl and

$-OR^{IV}$ with $R^{IV}$ being methyl, ethyl, propyl, methoxymethyl, allyl or β-dimethylaminoethyl,

$R^2$ is a hydrogen atom, a methyl or ethyl group and

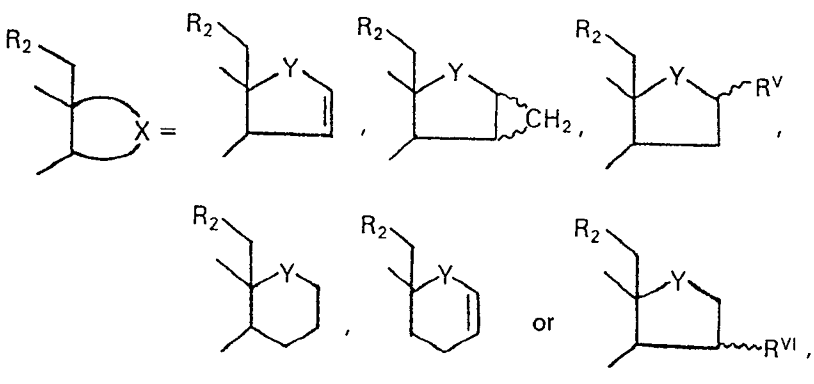

in which the wavy line ~~~ indicates that the substituent is in the α- or β-position and –Y– represents

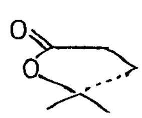 , 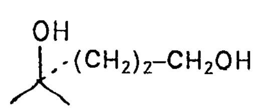 or 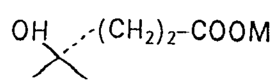

with M = K, Na or Li or

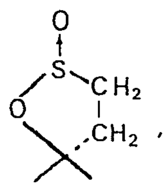 , 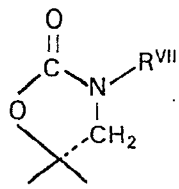 or 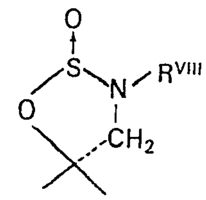

$R^V$ and $R^{VI}$ and $R^{VII}$ and $R^{VIII}$ in each case are alkyl of 1 to 4 carbon atoms, characterised in that a compound of the general formula II

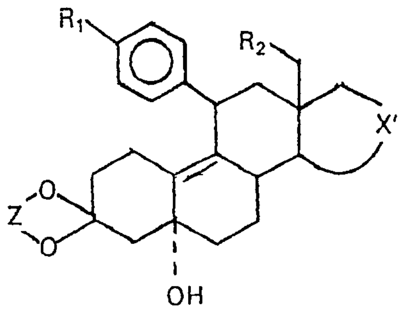

(II),

wherein

$R_1$ and $R_2$ have the meanings given in formula I,
Z represents an ethylene- or 2,2-dimethylpropylene-group and, in the case where
X' represents X as in formula I and Y represents

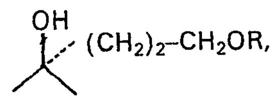

where R represents a hydrogen atom or a light separable group, for the preparation of compounds of formula I where
-Y- =

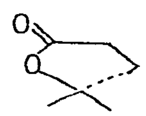 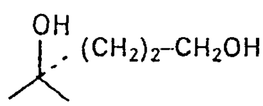 or 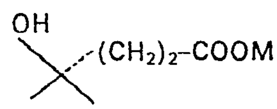

with M = K, Na or Li
with a weak acid or with an ion-exchange acid at a temperature of 0–100 °C and optionally oxidises the obtained 17α-(3-hydroxypropyl)-compound in a known way to the 17α-propionic acid lactone and optionally opens the lactone ring with alkali to the corresponding 17α-propionic acid alkali salt, and, in the case where X' is as X in formula I with Y having the meaning

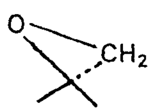

a) for the preparation of compounds of the formula I with -Y- =

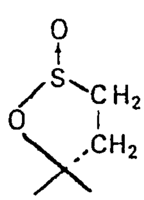

are treated with tert-butylmethylsulphoxide and alkyllithium in tetrahydrofuran, then with weak acids and with N-chloro- or N-bromosuccinimide,

b) for the preparation of compounds of formula I with Y =

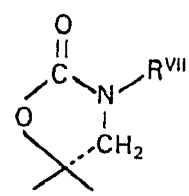

is reacted with alkylamine in the presence of an acid, then with carboxylic acid dialkyl ester in the presence of a base and joined with weak acid or is reacted with N-alkylurethane and potassium-tert. butylate in hexamethylphosphoric acid triamide and then with weak acid and

c) for the preparation of compounds of formula I with -Y- =

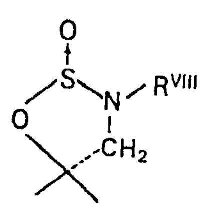

is treated with alkylamine in the presence of an acid, then with thionyl chloride in triethylamine and joined with weak acid.

**Claim for the contracting state: AT**

Process for the preparation of 11-estradienes of the general formula I

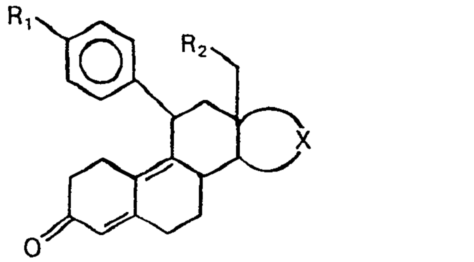

(I),

wherein

$R_1$ is 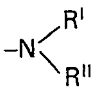 with $R^I$ and $R^{II}$ in each case being alkyl of 1 to 4 carbon atoms or $R^I$ and $R^{II}$ together with the N representing a 5- or 6- membered heterocyclic ring which besides the N can contain a further heteroatom N, O or S and the corresponding N-oxides,

$-SR^{III}$ with $R^{III}$ being methyl, ethyl or phenyl and

$-OR^{IV}$ with $R^{IV}$ being methyl, ethyl, propyl, methoxymethyl, allyl or β-dimethylaminoethyl,

$R^2$ is a hydrogen atom, a methyl or ethyl group and

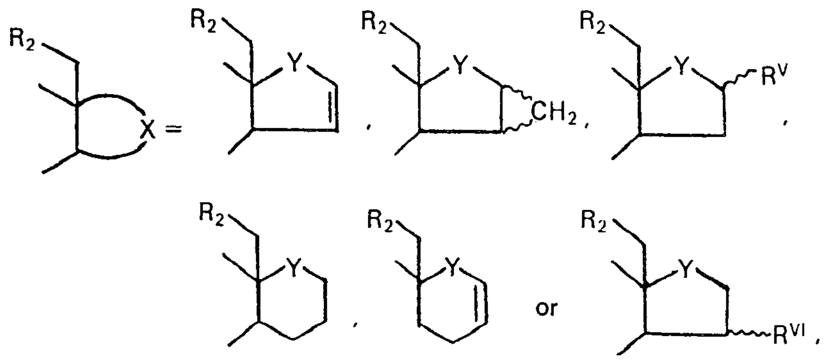

in which the wavy line 〰 indicates that the substituent is in the α- or β-position and –Y– represents

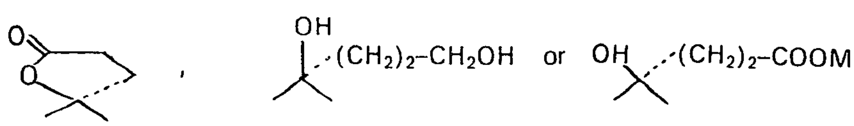

with M = K, Na or Li or

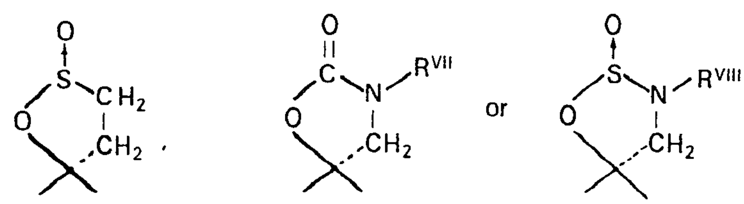

$R^V$ and $R^{VI}$ and $R^{VII}$ and $R^{VIII}$ in each case are alkyl of 1 to 4 carbon atoms, characterised in that a compound of the general formula II

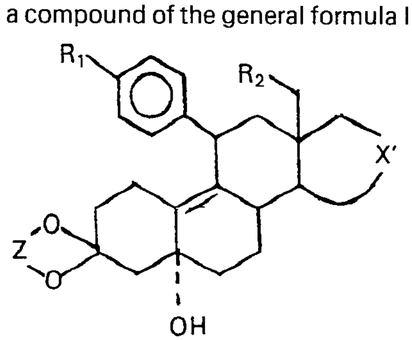

(II),

wherein

$R_1$ and $R_2$ have the meanings given in formula I,

Z represents an ethylene- or 2,2-dimethylpropylene-group and, in the case where

X' represents X as in formula I and Y represents

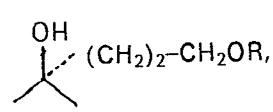

where R represents a hydrogen atom or a light separable group, for the preparation of compounds of formula I where

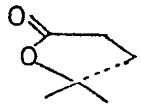

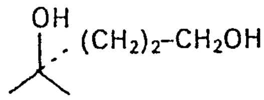

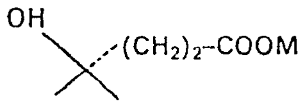

with M = K, Na or Li
with a weak acid or with an ion-exchange acid at a temperature of 0–100 °C and optionally oxidises the obtained 17α-(3-hydroxypropyl)-compound in a known way to the 17α-propionic acid lactone and optionally opens the lactone ring with alkali to the corresponding 17α-propionic acid alkali salt, and, in the case where X' is as X in formula I with Y having the meaning

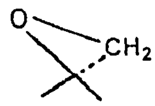

a) for the preparation of compounds of the formula I with –Y– =

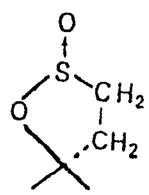

are treated with tert-butylmethylsulphoxide and alkyllithium in tetrahydrofuran, then with weak acids and with N-chloro- or N-bromosuccinimide,

b) for the preparation of compounds of formula I with Y =

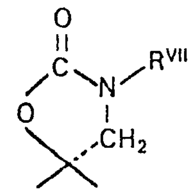

is reacted with alkylamine in the presence of an acid, then with carboxylic acid dialkyl ester in the presence of a base and joined with weak acid or is reacted with N-alkylurethane and potassium-tert.butylate in hexamethylphosphoric acid triamide and then with weak acid and

c) for the preparation of compounds of formula I with –Y– =

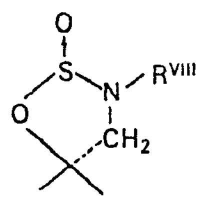

is treated with alkylamine in the presence of an acid, then with thionyl chloride in triethylamine and joined with weak acid.